# EUROPEAN PATENT APPLICATION

(11) **EP 3 067 365 A1**
(43) Date of publication of application: **14.09.2016**
(21) Application number: 14860721.1
(22) Date of filing: 10.10.2014
(51) Int. Cl.: C07K 14/64, C12N 15/16, C12N 15/70, C12N 15/81, C12N 1/21, C12N 1/19, A61K 38/22, A61P 9/00

(54) **HUMAN RELAXIN ANALOGUE, PHARMACEUTICAL COMPOSITION OF SAME, AND PHARMACEUTICAL APPLICATION OF SAME**

(30) Priority: 07.11.2013 CN 201310548221
(71) Applicant: Shanghai Hengrui Pharmaceutical Co. Ltd., Minhang District Shanghai 200245 (CN); Jiangsu Hengrui Medicine Co. Ltd., Jiangsu 222047 (CN)
(72) Inventor: ZHANG, Lianshan, Lianyungang Jiangsu 222047 (CN); LIU, Jiajian, Shanghai 200245 (CN); CAO, Guoqing, Shanghai 200245 (CN)
(74) Representative: Grünecker Patent- und Rechtsanwälte PartG mbB
(86) International application number: PCT/CN2014/088280
(87) International publication number: WO 2015/067113

(57) **Abstract**

Disclosed are a human relaxin analogue, polynucleotide for encoding the human relaxin analogue, a pharmaceutical composition of the human relaxin analogue, and a pharmaceutical application of the human relaxin analogue. Further disclosed is a derivative of the human relaxin analogue.

## Description

### FIELD OF THE INVENTION

The present disclosure relates to a novel human relaxin analog, and the pharmaceutical composition containing the same, and its use.

### BACKGROUND OF THE INVENTION

Relaxin (referred as RLX) is a polypeptide hormone secreted by mammal corpus luteum. Relaxin has a variety of physiological functions *in vivo,* including stretching pubic ligament, inhibiting uterine contractions, softening cervix, stimulating breast development and affecting galactosis. In 1926, relaxin for the first time was discovered by Frederick Hisaw in the study of pelvic girdle changes during pregnancy. Relaxin was considered as a double-stranded protein during 1970s and 1980s. The structure of relaxin is similar to that of insulin. It has been verified that relaxin is a member of the family of peptide hormones. Relaxin family *of Homa sapiens* is encoded by seven genes: RLN1, RLN2, RLN3 (also for INSL7), INSL3/RLF, INSL4/EPIL, INSL5/RIF2 and INSL6/RIF1. Most of relaxins in the human circulation are products encoded by RLN2 gene. The translation product of RLN2 is relaxin precursor, comprising (from N-terminal to C-terminal): 24 amino acid residues of signal peptide, 29 amino acid residues of chain B, 104 to 107 amino acid residues of linker, and about 24 amino acid residues of chain A.

It is clearly that relaxin not only plays an important role in pregnancy, but also exerts an effect on structure and function of vessel in non-pregnant animals. Relaxin has a wide range of biological effects, including homeostasis maintenance of internal environment during pregnancy and aging process of mammals, anti-inflammation, cardio-protection, dilation of blood vessels, promoting wound healing, and particularly anti-fibrosis. Heart diseases and kidney diseases caused by various factors ultimately lead to fibrosis, structural changes and loss of function. Therefore, effective inhibition of fibrosis is an important measure to retrieve the organ function. As a result, the anti-fibrotic effect of relaxin may be an effective action for anti-fibrotic therapy in future. More importantly, relaxin is produced by the heart, and exhibits heart protection and extracellular matrix modulation through local receptors. Relaxin has been successfully used to ameliorate cardiac fibrosis in various animal models. Thus, relaxin is expected to be useful in treatment of human heart fibrotic diseases (Xiaojun Du, Juan Zhou, Baker Heart Institute).

In the prior art, human relaxin (hRelaxin, wild-type) precursor is expressed in *E*. *coli;* and refolded after purification; digested by CPB and trypsin respectively in a two-step reaction; and then purified to obtain active product relaxin. Deficiencies in the prior art involve: protein refolding, two-step digestion and re-purification are required in the production process. Varied steps would greatly reduce yield and increase cost, as each step involves loss of protein. Furthermore, exogenous enzymes (CPB, trypsin, etc.) are costly, the quality of enzymes also affect the quality and yield of the final product.

Another drawback is that the expressed wild-type relaxin exhibits low activity, leading to increased amount of recombinant protein needed by treatment, and thereby an increased cost. Furthermore, low activity of expressed wild-type relaxin brings difficulty to further development of protein modification, since modification will damage the protein activity.

For the above-mentioned deficiencies of the prior art, the present disclosure designs a series of novel molecules by altering amino acid(s) of wild-type relaxin at specific site(s), and discovers novel human relaxin analogs which can excellently solve the above-mentioned problems. The new technical solution shows the following advantages:
1. Amino acid(s) alternation at particular site(s) of relaxin helps the analogs to be correctly folded in yeast cells, the c-peptides of chain A and chain B are removed by endogenous enzyme system of the cells in the process. Complete active molecules with physiological function are obtained and secreted extracellularly. The active molecules are obtained by one-step purification. As a result, the steps such as refolding, digestion and purification required in the prior art can be omitted.
2. Amino acid alternation at particular site(s) of relaxin makes the structure of relaxin analogs more suitable for intracellular folding, digestion and secretion. Furthermore, the biological activity of relaxin is improved. Thus, the amount of recombinant protein desired in unit dosage can be reduced, leading to lower cost. Meanwhile, the development of molecular modification would be much easier, due to the increased activity of such analogs.

Human relaxin analogs with specific sequences are available in present disclosure, via design (replacement or deletion of specific amino acid(s) in relaxin sequence, etc.). Protein folding and enzyme digestion of relaxin precursor can be realized in eukaryotic expression system (cell); and when the analog is secreted into the fermentation broth, the human relaxin analog of present disclosure is mature, intact and functional. Moreover, biological activity of relaxin analogs obtained through these particular designs is at least 2 times higher than that of wild-type.

### SUMMARY OF THE INVENTION

The present disclosure provides a human relaxin analog, comprising chain A and chain B, the amino acid sequence of the chain A and chain B are represented respectively by the following formula:
chain A: A₁LYSALANKCCHVGCTKRSLARFC (24 amino acid residues),
chain B: DSWMEEVIKLCGRB1₄LVRAQIAICGMSTWS (29 amino acid residues),
wherein A₁ is selected from the group consisting of Q, D, E, and W;
wherein B₁₄ is selected from the group consisting of E, D and N;
optionally, B₁ and B₂ are absent simultaneously; where A₁ is Q, B₁₄ is not E or D.

In one embodiment of the present disclosure, a human relaxin analog as described above is provided, comprising chain A and chain B, the amino acid sequences of the chain A and chain B are represented by the following formula, respectively:
chain A: A₁LYSALANKCCHVGCTKRSLARFC (24 amino acid residues)
chain B: DSWMEEVIKLCGRB₁₄LVRAQIAICGMSTWS (29 amino acid residues)
wherein A₁ is selected from the group consisting of Q, D, E, and W;
wherein B₁₄ is selected from the group consisting of E, D and N;
where A₁ is Q, B₁₄ is not E or D.

In one embodiment of the present disclosure, A₁ is D, E or W, preferably D.

In one embodiment of the present disclosure, A₁ is D, B₁₄ is D. In particular, one example is a human relaxin analog comprising SEQ ID NO: 134 (chain A) and SEQ ID NO: 135 (chain B) which are linked to each other by disulfide bond.

In one embodiment of the present disclosure, B₁₄ is D.

In one embodiment of the present disclosure, the human relaxin analog as described above, comprising the amino acid sequences of said chain A and chain B which are selected from the group consisting of:

| | |
|---|---|
| Chain B: DSWMEEVIKLCGRDLVRAQIAICGMSTWS | SEQ ID NO: 137 |
| chain A: DLYSALANKLCCHVGCTKRSLARFC | SEQ ID NO: 138; |
| | |
| chain B: DSWMEEVIKLCGRDLVRAQIAICGMSTWS | SEQ ID NO: 137 |
| chain A: QLYSALANKCCHVGCTKRSLARFC | SEQ ID NO: 135; |
| | |
| chain B: DSWMEEVIKLCGRDLVRAQIAICGMSTWS | SEQ ID NO: 137 |
| chain A: ELYSALANKCCHVGCTKRSLARFC | SEQ ID NO: 136; |
| | |
| chain B: DSWMEEVIKLCGRNLVRAQIAICGMSTWS | SEQ ID NO: 139 |
| chain A: QLYSALANKCCHVGCTKRSLARFC | SEQ ID NO: 135; |
| | |
| chain B: WMEEVIKLCGRDLVRAQIAICGMSTWS | SEQ ID NO: 140 |
| chain A: DLYSALANKCCHVGCTKRSLARFC | SEQ ID NO: 138; |
| | |
| chain B: DSWMEEVIKLCGRELVRAQIAICGMSTWS | SEQ ID NO: 134 |
| chain A: WLYSALANKCCHVGCTKRSLARFC | SEQ ID NO: 141; |
| and | |
| chain B: DSWMEEV1KLCGRELVRAQIAICGMSTWS | SEQ ID NO: 134 |
| chain A: DLYSALANKCCHVGCTKRSLARFC | SEQ ID NO: 138. |

In one embodiment of the present disclosure, the human relaxin analog defined above, wherein said chain B is linked to said chain A by linker sequence (referred to L in this disclosure), wherein the linker sequence has 1 to 15 amino acid residues in length, preferably 2 to 8 amino acid residues, the amino acid sequence of said linker sequence is selected from the group consisting of:

| | |
|---|---|
| L1: KR, | |
| L2: KRKPTGYGSRKKR, | SEQ ID NO: 27, |
| L3: KRKPTGYGSRKR, | SEQ ID NO: 28, |
| L4: KRGGGPRR, | SEQ ID NO: 29, |
| L5: KRGGGPKR, | SEQ 1D NO: 30, |
| L6: KRKPTGYGSKR, | SEQ ID NO: 31, and |
| L7: KRSLKR | SEQ ID NO: 32. |

In one embodiment of the present disclosure, provided is the human relaxin analog described above, wherein N terminal of said human Relaxin analog is linked to signal peptide sequence (referred to S in this disclosure), the signal peptide sequence has 4 to 15 amino acid residues in length, preferably 6 to 11 amino acid residues; more preferably, the amino acid sequence of said signal peptide sequence is selected from the group consisting of:

| | |
|---|---|
| S1: EEGEPK, | SEQ ID NO: 33, |
| S2: EEGEPKR, | SEQ ID NO: 34, and |
| S3: MKKNIAPLLKR | SEQ ID NO: 35. |

In one embodiment of the present disclosure, provided is an expression precursor, which is used for preparing the human relaxin analog described above, the amino acid sequence of said expression precursor is one or more sequence(s) selected from the group consisting of SEQ ID NO: 1 to SEQ ID NO: 26.

**Table 1. Summary of Expression precursor of human relaxin analog**

| Example | Amino acid sequence of human relaxin analog expression precursor (SEQ ID NO) | abbreviation |
|---|---|---|
| relaxin 800800 | DSWMEEVIKLCGRELVRAQIAICGMSTW SKRQLYSALANKCCHVGCTKRSLARFC (SEQ ID NO: 1) | B(wt)-L1-A(wt) |
| relaxin 800801 | DSWMEEVIKLCGRELVRAQIAICGMSTW SKRSLKRQLYSALANKCCHVGCTKRSLA RFC (SEQ ID NO: 2) | B(wt)-L7-A(wt) |
| relaxin 800802 | DSWMEEVIKLCGRELVRAQIAICGMSTW SKRKPTGYGSRKKRQLYSALANKCCHV GCTKRSLARFC (SEQ ID NO: 3) | B(wt)-L2-A(wt) |
| relaxin 800802-1 | DSWMEEVIKLCGRDLVRAQIAICGMSTW SKRKPTGYGSRKKRDLYSALANKCCHV GCTKRSLARFC (SEQ ID NO: 4) | D^{B14}-L2-D^{A1} |
| relaxin 800803 | DSWMEEVIKLCGRELVRAQIAICGMSTW SKRKPTGYGSRKRQLYSALANKCCHVG CTKRSLARFC (SEQ ID NO: 5) | B(wt)-L3-A(wt) |
| relaxin 800803-1 | DSWMEEVIKLCGRDLVRAQIAICGMSTW SKRKPTGYGSRKRDLYSALANKCCHVG CTKRSLARFC (SEQ ID NO: 6) | D^{B14}-L3-D^{A1} |
| relaxin 800805 | DSWMEEVIKLCGRELVRAQIAICGMSTW SKRGGGPRRQLYSALANKCCHVGCTKRS LARFC (SEQ ID NO: 7) | B(wt)-L4-A(wt) |
| relaxin 800805-1 | DSWMEEVIKLCGRDLVRAQIAICGMSTW SKRGGGPRRDLYSALANKCCHVGCTKRS LARFC (SEQ ID NO: 8) | D^{B14}-L4-D^{A1} |
| relaxin 800806 | DSWMEEVIKLCGRELVRAQIAICGMSTW SKRGGGPKRQLYSALANKCCHVGCTKR SLARFC (SEQ ID NO: 9) | B(wt)-L5-A(wt) |
| relaxin 800806-1 | DSWMEEVIKLCGRDLVRAQIAICGMSTW SKRGGGPKRDLYSALANKCCHVGCTKR SLARFC (SEQ ID NO: 10) | D^{B14}-L5-D^{A1} |
| relaxin 800808 | EEGEPKDSWMEEVIKLCGRELVRAQIAIC GMSTWSKRQLYSALANKCCHVGCTKRS LARFC (SEQ ID NO: 11) | S1-B(wt)-L1-A(wt) |
| relaxin 800808-1 | EEGEPKDSWMEEVIKLCGRDLVRAQIAIC GMSTWSKRDLYSALANKCCHVGCTKRS LARFC (SEQ ID NO: 12) | S1-DB¹⁴-L1-D^{A1} |
| relaxin 800809 | EEGEPKRDSWMEEVIKLCGRELVRAQIAI CGMSTWSKRQLYSALANKCCHVGCTKR SLARFC (SEQ ID NO: 13) | S2-B(wt)-L1-A(wt) |
| relaxin 800809-1 | EEGEPKRDSWMEEVIKLCGRDLVRAQIAI CGMSTWSKRDLYSALANKCCHVGCTKR SLARFC (SEQ ID NO: 14) | S2-D^{B14}-L1-D^{A1} |
| relaxin 800810 | DSWMEEVIKLCGRDLVRAQIAICGMSTW SKRQLYSALANKCCHVGCTKRSLARFC (SEQ ID NO: 15) | D^{B14}-L1-A(wt) |
| retaxin 800810-1 | DSWMEEVIKLCGRDLVRAQIAICGMSTW SKRDLYSALANKCCHVGCTKRSLARFC (SEQ ID NO: 16) | D³¹⁴-L1-D^{A1} |
| relaxin 800811 | DSWMEEVIKLCGRDLVRAQIAICGMSTW SKRKPTGYGSKRQLYSALANKCCHVGCT KRSLARFC | D^{B14}-L6-A(wt) |
| | (SEQ ID NO: 17) | |
| relaxin 800813 | DSWMEEVIKLCGRDLVRAQIAICGMSTW SKRKPTGYGSKRELYSALANKCCHVGCT KRSLARFC (SEQ ID NO: 18) | D^{B14}-L6-E^{A1} |
| relaxin 800814 | DSWMEEVIKLCGRDLVRAQIAICGMSTW SKRKPTGYGSKRDLYSALANKCCHVGCT KRSLARFC (SEQ ID NO: 19) | D^{B14}-L6-D^{A1} D^{B14}-L6-D^{A1} |
| relaxin 800816 | DSWMEEVIKLCGRNLVRAQIAICGMSTW SKRKPTGYGSKRQLYSALANKCCHVGCT KRSLARFC (SEQ ID NO: 20) | N^{B14}-L6-A(wt) |
| relaxin 800847Y | DSWMEEVIKLCGRELVRAQIAICGMSTW SKRKPTGYGSKRDLYSALANKCCHVGCT KRSLARFC (SEQ ID NO: 21) | B(wt)-L6-D^{A1} |
| relaxin 800851Y | WMEEVIKLCGRDLVRAQIAICGMSTWSK RKPTGYGSKRDLYSALANKCCHVGCTK RSLARFC (SEQ ID NO: 22) | D^{B14}-L6-D^{A1} |
| relaxin 800828 | MKKNIAFLLKRDSWMEEVIKLCGRELVR AQIAICGMSTWSKRKPTGYGSRKKRQLY SALANKCCHVGCTKRSLARFC (SEQ ID NO: 23) | S3-B(vvt)-L2-A(wt) |
| relaxin 800843 | MKKNIAFLLKRDSWMEEVIKLCGRELVR AQIAICGMSTWSKRKPTGYGSRKKRWLY SALANKCCHVGCTKRSLARFC (SEQ ID NO: 24) | S3-B(wt)-L2-W^{A1} |
| relaxin 800847 | MKKNIAFLLKRDSWMEEVIKLCGRELVR AQIAICGMSTWSKRKPTGYGSKRDLYSA LANKCCHVGCTKRSLARFC (SEQ ID NO: 25) | S3-B(wt)-L6-D^{A1} |
| relaxin 800851 | MKKNIAFLLKRWMEEVIKLCGRDLVRA QIAICGMSTWSKRKPTGYGSKRDLYSAL ANKCCHVGCTKRSLARFC (SEQ ID NO: 26) | S3-Del^{B1,B2}-D ^{B14}-L6-D^{A1} |

The present disclosure further provides human relaxin analog derivatives obtained by PEG-modification of human relaxin analogs described above. In other words, the human relaxin analogs described above are modified by PEG molecule. In some embodiments, the molecular weight of PEG is 5 to 100 KDa; in some embodiments, 10 to 80 KDa; in some embodiments, 15 to 45 KDa; in some embodiments, 20 to 40 KDa; in some embodiments, PEG molecule is a branched-chain type or a linear- chain type.

The present disclosure further provides a polynucleotide encoding expression precursor of human relaxin analog described above. The polynucleotide is selected from DNA or RNA. It is appreciated by those skilled in the art that the complementary sequence of the polynucleotide is also within the scope of this disclosure.

The present disclosure further provides an expression vector comprising the polynucleotide as described above.

The present disclosure further provides a host cell transformed with the expression vector as described above. In some embodiments, the host cell is bacterial, in some particular embodiments, the host cell is *E. coli;* in other embodiments, the host cell is yeast, in some embodiments, the host cell is *Pichia pas*/*oris.*

The present disclosure further provides a pharmaceutical composition which comprises or consists of the following components:
a) one or more human relaxin analog(s) as described above, and/or one or more human relaxin analog derivative(s) as described above, and
b) one or more pharmaceutically acceptable carriet(s), diluent(s) or excipient(s).

The present disclosure further provides an injectable solution of human relaxin analog or derivative thereof, the injectable solution contains a dissolved form or dissoluble form of the pharmaceutical composition as described above. It should be understood that the dry powder or lyophilized powder form of the injectable solution is also encompassed within the scope of this disclosure.

The present disclosure further provides the use of said human relaxin analog as described above, the human relaxin analog derivative as described above, or the pharmaceutical composition as described above, or the injectable solution described above, in preparation of a medicament for the treatment or prevention offibrotic disease or cardiovascular disease, for reference, Cardiovascular effects of relaxin; from basic science to clinical therapy, Nat.Rev.Cardiol.7,48-58(2010); Relaxin decreases renal interstitial fibrosis and slows progression of renal disease, Kindney International, Vol. 59(2001), pp. 876-882.

The present disclosure further provides a method for treating or preventing fibrotic disease or cardiovascular disease, the method comprises a step of administering to a subject in need thereof a therapeutically effective amount of human relaxin analog as described above or the derivative thereof, or the pharmaceutical composition as described above.

### DETAILED DESCRIPTION OF THE INVENTION

### Terms

For better understanding of the present disclosure, certain technical and scientific terms are specifically defined below. Unless specifically defined elsewhere in this document, all other technical and scientific terms used herein have the meaning commonly understood by one of ordinary skill in the art to which this disclosure belongs.

As used herein, the single-letter code and the three-letter code for amino acids are as described in J. Biol. Chem, 243, (1968) p3558.

"Administered", "administration", "treatment" and "treating", as it applies to an animal, human, experimental subject, cell, tissue, organ, or biological fluid, refers to contact of an exogenous pharmaceutical, therapeutic, diagnostic agent, or composition to the animal, human, subject, cell, tissue, organ, or biological fluid. "Administered", "administration", "treatment" and "treating" refer, e.g., to therapeutic, pharmacokinetic, diagnostic, research, and experimental methods. Treatment of a cell encompasses contact of a reagent to the cell, as well as contact of a reagent to a fluid, where the fluid is in contact with the cell. "Administered", "administration", "treatment" and "treating" also means *in vitro* and *ex vivo* treatments of a cell, by a reagent, diagnostic, binding composition, or by another cell. "Treatment" as it applies to a human, veterinary, or research subject, refers to therapeutic treatment, prophylactic or preventative measures, research and diagnostic applications.

"Treat" or "treating" means to internally or externally administer a therapeutic agent, such as a composition containing any of the binding compounds of the present disclosure, to a patient wtih one or more disease symptoms for which the agent has known therapeutic activity. Typically, the therapeutic agent is administered in an amount effective to alleviate one or more disease symptoms in the treated patient or population, regardless of how does the effective amount take effect, either by inducing the regression of such symptom(s) or by preventing such symptom(s) from developing into any clinically measurable degree. The amount of a therapeutic agent that is effective to alleviate any particular disease symptom (also referred to as the "therapeutically effective amount") may vary according to factors such as disease state, age, and weight of the patient, as well as the ability of the drug in the aspect of eliciting a desired response in the patient. Whether a disease symptom has been alleviated can be assessed by any clinical measurement typically used by physicians or other skilled healthcare providers to assess the severity or progression status of that symptom. Though the embodiments of the present disclosure (e.g., a treatment method or manufacture product) may vary in alleviating the target disease symptom(s) among patients, the embodiments should alleviate the target disease symptom(s) in patients with statistical significance, as determined by any statistical test known in the art, such as the Student's t-test, the chi square test, the U-test according to Mann and Whitney, the Kruskal-Wallis test (H-test), Jonckheere-Terpstra-test and the Wilcoxon-test.

Unless stated clearly elsewhere in this document, relaxin or human relaxin used herein is human relaxin RLN2, which comprises a full length sequence or a partial sequence having biological activity. Human relaxin contains chain A and chain B which are combined together via disulfide bond(s). The sequence description of human relaxin used herein refers to GenBank accession number: EAW58770. Relaxin 800828 is wild-type and used as positive control in this disclosure.

From the amino-terminus to carboxy-terminus, the human relaxin analog precursor according to present disclosure comprises: a signal peptide sequence, chain B or mutant thereof, linker, chain A or mutant thereon Wherein, the length of the signal peptide sequence is 4 to 15 amino acid residues, preferably 6 to 11 amino acid residues; the length of the chain B or variant thereof is 29 amino acid residues; the length of linker sequence is 1 to 15 amino acid residues, preferably 2 to 8 amino acid residues; and the length of chain A or variant thereof is about 24 amino acid residues.

Mutant used herein refers to amino acid modifications, replacements, substitutions, or displacements in the sequence. Preferable mutation in chain B is replacement of E at the 14^{th} amino acid residue (B₁₄ for short) with D, and replacement ofQ at the 1^{st} amino acid residue of chain A (referred to as A1) with D.

Sequence abbreviations used herein, Ln represents some linker sequences (e.g. L1 to L6) in this disclosure, Sn indicates certain signal peptide sequence (e.g., S1, S2, S3) of this disclosure; D^{A1} shows that the mutation at the 1^{st} amino acid of chain A is D, D^{B14} represents that the mutation at the 14^{th} amino acid of chain B is D, Del^{B1,B2} represents the absence of the 1^{st} and 2^{nd} amino acids of chain B; A(wt) represents wild type chain A without any mutation, B(wt) represents wild type chain B without any mutation.

"Conservative modificationl" or "conservative substitution" refers to substitution of amino acid(s) in a protein by other amino acid residue(s) having similar characteristics (e.g. charge, side-chain size, hydrophobicity/hydrophilicity, backbone conformation and rigidity, etc.), such that the changes can frequently be made without altering the biological activity of the protein. Those of skill in this art recognize that, in general, single amino acid substitution in non-essential region of a polypeptide does not substantially alter biological activity (see, e.g., Watson et al. (1987) Molecular Biology of the Gene, The Benjamin/Cummings Pub. Co., p. 224, 4th Ed.). In addition, substitutions for structurally or functionally similar amino acid residues are less likely to disrupt biological activity.

"Effective amount" encompasses an amount sufficient to ameliorate or prevent a symptom or sign of the medical condition. Effective amount also means an amount sufficient to allow or facilitate diagnosis. An effective amount for a particular subject may vary depending on factors such as the condition being treated, the overall health of the patient or veterinary subject, the administration method, route and dose of administration and the severity of side effects. An effective amount can be the maximal dose or dosing protocol that avoids significant side effects or toxic effects.

"Exogenous" refers to substances that are produced outside an organism, cell, or human body. "Endogenous" refers to substances that are produced within a cell, organism, or human body.

As used herein, the expressions "cell", "cell line" and "cell culture" are used interchangeably, and all such designations include progeny. Thus, the words "transformant" and "transformed cell" include the primary subject cell and cultures derived therefrom, regardless of the number of passage. It is also understood that all progeny may not be precisely identical in DNA content, due to deliberate or inadvertent mutations. Mutant progeny that have the same function or biological activity as that of originally transformed cell are included. Where distinct designations are intended, it will be clear from the context.

As used herein, "polymerase chain reaction" or "PCR" refers to an amplification procedure or technique as described in e.g. U.S. Pat. No. 4,683,195. Generally, sequence information from the ends of the region of interest or beyond needs to be available, such that oligonucleotide primers can be designed; these primers will be identical or similar in sequence to opposite strands of the template to be amplified. The 5' terminal nucleotides of the two primers can coincide with the ends of the amplified material. PCR can be used to amplify specific RNA sequences, specific DNA sequences from total genomic DNA, and cDNA transcribed from total cellular RNA, bacteriophage or plasmid sequences, etc. See generally Mullis et al. (1987) Cold Spring Harbor Symp. Quant. Biol. 51:263; Erlich, ed., (1989) PCR TECHNOLOGY (Stockton Press, N.Y.). As used herein, PCR is considered to be one, but not the only, example of a nucleic acid polymerase reaction method for amplifying a nucleic acid test sample, such method comprises the use of a known nucleic acid as a primer and a nucleic acid polymerase to amplify or generate a specific portion of nucleic acid.

"Optional" or "optionally" means that the event or situation that follows may but does not necessarily occur.

"Pharmaceutical composition" refers to a mixture comprising one or more analoge(s) or precursor(s) according to present disclosure, and additional chemical components, wherein said additional components are such as physiologically/pharmaceutically acceptable carriers and excipients. Said additional component aims at promoting the administration to an organism, facilitating the absorption of the active ingredient and thereby exerting a biological effect.

The transformation process of host cell stated in this disclosure is well known to those of skill in the art. The obtained transformant can be cultured by a conventional method, and it can express a polypeptide which is encoded by a gene of the present disclosure. The culture medium used herein is selected from various conventional culture mediums depending on the host cell used. The host cells are cultured under a suitable condition. Human relaxin analogs released from the product expressed by the precursor are available to chemical and/or enzymatic methods well known to those skilled in the art, such as using trypsin, carboxypeptidase B, lysine endopeptidase C and etc.

### Examples

### Example 1 Cloning and expression of recombinant human relaxin 800800

### 1. Construction of recombinant human relaxin 800800 expression vector

DNA sequence of codon-optimized human relaxin 800800 was synthesized by Overlap PCR method. Six single-stranded DNA fragments synthesized by Invitrogen Company were used as synthetic primers, sequences were as follows:
800-primer 1
   CTCGAGGATTCTTGGATGGAAGAAGTTATTAAGT SEQ ID NO: 36
800-primer 2 
CAATTTGAGCTCTAACCAATTCTCTACCACACAACTTAATAACTTCTTC
CATCCAAGAA SEQ ID NO: 37
800-primer 3 
AGAGAATTGGTTAGAGCTCAAATTGCTATTTGTGGTATGTCTACTTGGT
CTAAGAGACA SEQ ID NO: 38
800-primer 4 
ATGACAACACTTGTTAGCCAAAGCAGAGTACAATTGTCTCTTAGACCA
AGTAGACATAC SEQ ID NO: 39
800-primer 5 
CTTTGGCTAACAAGTGTTGTCATGTTGGTTGTACTAAGAGATCTTTGGC
TAGATTTTGT SEQ ID NO: 40
800-primer 6 GAATTCTTAACAAAATCTAGCCAAAGATCTCTTA

SEQ ID NO: 41 Relaxin was synthesized by KOD plus kit (TOYOBO, Cat. KOD-201), and the reaction was performed by two-step PCR.

PCR step 1, 25µL reaction system: 2.5µL of 10×KOD buffer, 2.5µL of 2mM dNTPs, primers 1, 2, 3, 4, 5, 6 (10µM) each 1µL, 0.5µL of KOD plus, 1µL of 25mM MgSO4,12.5 µL of ddH₂O.

Reaction program: 94°C 5 minutes; 94 °C 30 seconds, 60°C 30 seconds, 68 °C 30 seconds, for 30 cycles of amplification; then 68 °C 30 minutes to terminate PCR amplification process.

PCR step 2, 25µL reaction system: 2.5µL of 10×KOD buffer, 2.5µL of 2mM dNTPs, primers 1 and 6 (10µM) each 1µL, 1µL of round 1 PCR product, 0.5µL of KOD plus, I µL of 25mM MgSO4,15.5µL of ddH₂O.

Reaction program: 94 °C 5 minutes; 94 °C 30 seconds, 68 °C 60 seconds, for 30 cycles of amplification; then 68 °C 10 minutes to terminate PCR amplification process.

DNA sequences synthesized by PCR and pPIC9K expression vector (Invitrogen, Cat. K1750-01) were respectively digested using EcoR I/Xho I (New England Biolabs, Cat. R0101S/R0146V), obtained fragments of interest were recovered by 1.2% agarose gel, linked by T4 DNA ligase (New England Biolabs, Cat. M0202V), and transformed into DH5α competent cells (Tiangen, Cat. CB101-02). Positive clones were picked, and sequenced by Invitrogen. DNA sequence of 800 precursor is as follows:

GATTCTTGGATGGAAGAAGTTATTAAGTTGTGTGGTAGAGAATTGGTTA
GAGCTCAAATTGCTATTTGTGGTATGTCTACTTGGTCTAAGAGACAATTGTA
CTCTGCTTTGGCTAACAAGTGTTGTCATGTTGGTTGTACTAAGAGATCTTTG
GCTAGATTTTGTTAA SEQ ID NO: 42

Amino acid sequence coded by DNA above is as follows:

DSWMEEVIKLCGRELVRAQIAICGMSTWSKRQLYSALANKCCHVGCTKRS
LARFC SEQ ID NO: 1.

### 2. Transformation of the recombinant human relaxin 800800

Five ta10 µg of relaxin 800800 expression vector obtained from the above steps was linearized with Sall (Takata, Cat. D1080a), followed by addition of 1/10 volume of 3M sodium acetate, 2 volumes of anhydrous ethanol, the mixture was placed in -20°C for 2hr. The mixture was centrifuged at high speed (13,000rpm) for 5min, the supernatant was removed, and the pellet was rinsed with 75% ethanol for 2 times, dried invertedly, dissolved in 10µL of ddH₂O. The linearized plasmid and 80µL, of electroporation competent cells (Pichia GS115, Invitrogen, Cat. K1750-01) were mixed and loaded onto the electroporation cuvette(Bio Rad, Cat. 1652086) for 5 minutes in an ice bath. The electroporation was performed with parameters of the electroporation device (Bio Rad Micropulser) of 2 kV, 25Ω, 200 uF. After that, 1 ml of ice-bath D-sorbitol (Bioengineering Co., Ltd.) was added rapidly and mixed; and100 to 300µl of the mixture was spread onto the MD plate, and cultured for 3 days at 30°C until colonies were observed.

### 3. G418 selection of recombinant human relaxin 800800 expression clone

Colony on MD culture plate was eluted with 3ml of YPD medium, re-suspended, and the concentration of re-suspended cells was measured (1 OD₆₀₀ = 5×10⁷ cell/ml) by a spectrophotometer (Beckman, DU800). The 1×10⁵ of cells were spread on the YPD plate containing 4mg/ml of G418 (GIBCO, Cat. 11811-031), and cultured for 5 days at 30°C until colonies were observed.

### 4. Inducible expression of recombinant human relaxin 800800

Single colony was picked from YPD plate, placed in 4mL of BMGY medium and cultured overnight at 30°C, 250 rpm. The medium was detected for OD₆₀₀ value the next day, the value should be between 2 and 6. Cells were collected by centrifuge (Beckman Coulter) at a low speed (1,500g) for 5min at room temperature, and resuspended in BMMY medium until OD6⁰⁰ was 1.0. 1/200 volume of 100% methanol (final concentration of 0.5%) was added, and incubated at 28 °C, 250 rpm for 72hr, 1/200 of 100% methanol was supplemented per 24hr. After induction, the mixture was centrifuged at low-speed (1,500g) and the supernatant was collected. The protein expression was detected by electrophoresis SDS-PAGE (Invitrogen, Cat. No. 456-1083), preferred clones were selected for the subsequent fermentation step.

Sequences of resulting recombinant human relaxin 800800 (WT, wild-type) were as follows:

| | |
|---|---|
| chain B (WT) DSWMEEVIKLCGRELVRAQIAICGMSTWS | SEQ ID NO: 134 |
| chain A (WT) QLYSALANKCCHVGCTKRSLARPC | SEQ ID NO: 135. |

### Example 2 Cloning and expression of recombinant human relaxin 800801

### 1. Construction of recombinant human relaxin 800801 expression vector

Relaxin 800801 was synthesized by PCR site-directed mutagenesis. Two single-stranded DNA fragments synthesized by Invitrogen Company were used as site-directed mutagenesis primers, sequences were as follows:
801- Primer 1
   GTATGTCTACTTGGTCTAAGAGATCTTTGAAGAGACAATTGTACTC
   SEQ ID NO: 43
801- Primer 2
   GAGTACAATTGTCTCTTCAAAGATCTCTTAGACCAAGTAGACATAC
   SEQ ID NO: 44

Relaxin 800800 carrier was used as a template of PCR site-directed mutagenesis, KOD plus kit (TOYOBO, Cat KOD-201) 25µL reaction system: 2.5µL of 10×KOD buffer, 2.5µL of 2mM dNTPs, primers 1, 2 (10µM) each 1µL, 0,5µL of KOD plus, 1µL of 25mM MgSO4,16.5 µL of ddH₂O. Reaction program: 94°C 5 minutes; 94°C 30 seconds, 60°C; 30 seconds, 68°C 12 minutes, for 30 cycles of amplification; then 68°C 12 minutes to terminate PCR amplification process. PCR product was digested for 5 hours by direct addition of 1µL of endonuclease DpnI (NEB Cat. R0176L), and transformed into DH5α competent cells (Tiangen, Cat. CB101-02). Positive clones were picked, and sequenced by Invitrogen. DNA Sequence of 801 precursor is as follows:

GATTCTTGGATGGAAGAAGTTATTAAGTTGTGTGGTAGAGAATTGGTTA
GAGCTCAAATTGCTATTTGTGGTATGTCTACTTGGTCTAAGAGATCTTTGAA
GAGACAATTGTACTCTGCTTTGGCTAACAAGTGTTGTCATGTTGGTTGTACT
AAGAGATCTTTGGCTAGATTTTGTTAA SEQ ID NO: 45

Amino acid sequence coded by DNA above is as follows:

DSWMEEVIKLCGRELVRAQIAICGMSTWSKRSLKRQLYSALANKCCHVGC
TKRSLARFC SEQ ID NO: 2

### 2. Transformation, screening and inducible expression of the recombinant human relaxin 800801

Procedure for transformation, screening and inducible expression of the recombinant human relaxin 800801 was the same as that described in Example 1.

Sequences of resulting recombinant human relaxin 800801 (WT, wild-type) were as follows:

| | |
|---|---|
| chain B (WT) DSWMEEVIKLCGRELVRAQIAICGMSTWS | SEQ ID NO: 134 |
| chain A (WT) QLYSALANKCCHVGCTKRSLARFC | SEQ ID NO: 135. |

### Example 3 Cloning and expression of recombinant human relaxin 800802

### 1. Construction of recombinant human relaxin 800802 expression vector

Relaxin 800802 was synthesized by PCR site-directed mutagenesis. Two single-stranded DNA fragments synthesized by Invitrogen Company were used as site-directed mutagenesis primers, sequences were as follows:
802- Primer 1 
GTCTACTTGGTCTAAGAGAAAGCCAACTGGTTACGGTTCTAGAAAGAA
GAGACAATTGTACTCTGC SEQ ID NO: 46
802- Primer 2

GCAGAGTACAATTGTCTCTTCTTTCTAGAACCGTAACCAGTTGGCTTTC
TCTTAGACCAAGTAGAC SEQ ID NO: 47

Retaxin 800801 carrier was used as a template of PCR site-directed mutagenesis, KOD plus kit (TOYOBO, Cat KOD-201) 25µL reaction system: 2.5µL of 10×KOD buffer, 2.5µL of 2mM dNTPs, primers 1, 2 (10µM) each 1µL, 0.5µL of KOD plus, 1µL of 25mM MgSO4,16.5 µL of ddH₂O. Reaction program: 94°C 5 minutes; 94 °C 30 seconds, 60°C 30 seconds, 68°C 12 minutes, for 30 cycles of amplification; then 68°C 12 minutes to terminate PCR amplifcation process. PCR product was digested for 5 hours by direct addition of 1µL of endonuclease Dpnl (NEB Cat. R0176L), and transformed into DH5α competent cells (Tiangen, Cat. CB101-02). Positive clones were picked, and sequenced by Invitrogen. DNA Sequence of 802 precursor is as follows:

GATTCTTGGATGGAAGAAGTTATTAAGTTGTGTGGTAGAGAATTGGTTA
GAGCTCAAATTGCTATTTGTGGTATGTCTACTTGGTCTAAGAGAAAGCCAAC
TGGTTACGGTTCTAGAAAGAAGAGACAATTGTACTCTGCTTTGGCTAACAA
GTGTTGTCATGTTGGTTGTACTAAGAGATCTTTGGCTAGATTTTGTTAA
SEQ ID NO: 48

Amino acid sequence coded by DNA above is as follows:

DSWMEEVIKLCGRELVRAQIAICGMSTWSKRKPTGYGSRKKRQLYSALAN
KCCHVGCTKRSLARFC SEQ ID NO: 3.

### 2. Transformation, screening and inducible expression of the recombinant human relaxin 800802

Procedure for transformation, screening and inducible expression of the recombinant human relaxin 800802 was the same as that described in Example 1.

Sequences of resulting recombinant human relaxin 800802 (WT, wild-type) were as follows:

| | |
|---|---|
| chain B (WT) DSWMEEVIKLCGRELVRAQIAICGMSTWS | SEQ ID NO: 134 |
| chain A (WT) QLYSALANKCCHVGCTKRSLARFC | SEQ ID NO: 135. |

### Example 4 Cloning and expression of recombinant human relaxin 800802-1

### 1. Construction of recombinant human relaxin 800802-1 expression vector

Relaxin 800802-1 was synthesized by PCR site-directed mutagenesis. Four single-stranded DNA fragments synthesized by Invitrogen Company were used as site-directed mutagenesis primers, sequences were as follows:
802-1- Primer 1
   GTTATTAAGTTGTGTGGTAGAGATTTGGTTAGAGCTCAAATTG
   SEQ ID NO: 49
802-1- Primer 2
   CAATTTGAGCTCTAACCAAATCTCTACCACACAACTTAATAAC
   SEQ ID NO: 50
802-1- Primer 3 GTTCTAGAAAGAAGAGAGATTTGTACTCTGCTTTGG
   SEQ ID NO: 51
802-1- Primer 4 CCAAAGCAGAGTACAAATCTCTCTTCTTTCTAGAAC
   SEQ ID NO: 52

Relaxin 800802 carrier was used as a template of PCR site-directed mutagenesis, mutation process was the same as that described in Example 2. DNA Sequence of 800802-1 precursor is as follows:

GATTCTTGGATGGAAGAAGTTATTAAGTTGTGTGGTAGAGATTTGGTTA
GAGCTCAAATTGCTATTTGTGGTATGTCTACTTGGTCTAAGAGAAAGCCAAC
TGGTTACGGTTCTAGAAAGAAGAGAGATTTGTACTCTGCTTTGGCTAACAAG
TGTTGTCATGTTGGTTGTACTAAGAGATCTTTGGCTAGATTTTGTTAA
SEQ ID NO: 53

Amino acid sequence coded by DNA above is as follows:

DSWMEEVIKLCGRDLVRAQIAICGMSTWSKRKPTGYGSRKKRDLYSALA
NKCCHVGCTKRSLARFC SEQ ID NO: 4.

### 2. Transformation, screening and inducible expression of the recombinant human relaxin 800802-1

Procedure for transformation, screening and inducible expression of the recombinant human relaxin 800802-1 was the same as that described in Example 1.

Sequences of resulting recombinant human relaxin analog 800802-1 were as follows:

| | |
|---|---|
| chain B (D^{B14}) DSWMEEVIKLCGRDLVRAQIAICGMSTWS | SEQ ID NO: 137 |
| chain A (D^{A1}) DLYSALANKCCHVGCTKRSLARFC | SEQ ID NO: 138. |

### Example 5 Cloning and expression of recombinant human relaxin 800803

### 1. Construction of recombinant human relaxin 800803 expression vector

Relaxin 800803 was synthesized by PCR site-directed mutagenesis. Two single-stranded DNA fragments synthesized by Invitrogen Company were used as site-directed mutagenesis primers, sequences were as follows:
803- Primer 1 
GTCTACTTGGTCTAAGAGAAAGCCAACTGGTTACGGTTCTAGAAAGAG
ACAATTGTACTCTGC SEQ ID NO: 54
803- Primer 2

GCAGAGTACAATTGTCTCTTTCTAGAACCGTAACCAGTTGGCTTTCTCT
TAGACCAAGTAGAC SEQ ID NO: 55

Relaxin 800801 carrier was used as a template of PCR site-directed mutagenesis, mutation process was the same as that described in Example 2. DNA Sequence of 800803 precursor is as follows:

GATTCTTGGATGGAAGAAGTTATTAAGTTGTGTGGTAGAGAATTGGTTA
GAGCTCAAATTGCTATTTGTGGTATGTCTACTTGGTCTAAGAGAAAGCCAAC
TGGTTACGGTTCTAGAAAGAGACAATTGTACTCTGCTTTGGCTAACAAGTGT
TGTCATGTTGGTTGTACTAAGAGATCTTTGGCTAGATTTTGTTAA SEQ ID
NO: 56

Amino acid sequence coded by DNA above is as follows:

DSWMEEVIKLCGRELVRAQIAICGMSTWSKRKPTGYGSRKRQLYSALANK
CCHVGCTKRSLARFC SEQ ID NO: 5.

### 2. Transformation, screening and inducible expression of the recombinant human retaxin 800803

Procedure for transformation, screening and inducible expression of the recombinant human relaxin 800803 was the same as that described in Example 1.

Sequences of resulting recombinant human relaxin 800803 (WT, wild-type) were as follows:

| | |
|---|---|
| chain B (WT) DSWMEEVIKLCGRELVRAQIAICGMSTWS | SEQ ID NO: 134 |
| chain A (WT) QLYSALANKCCHVGCTKRSLARFC | SEQ ID NO: 135. |

### Example 6 Cloning and expression of recombinant human relaxin 800803-1

### 1. Construction of recombinant human relaxin 800803-1 expression vector

Relaxin 800803-1 was synthesized by PCR site-directed mutagenesis. Four single-stranded DNA fragments synthesized by Invitrogen Company were used as site-directed mutagenesis primers, sequences were as follows:
803-1- Primer 1
   GTTATTAAGTTGTGTGGTAGAGATTTGGTTAGAGCTCAAATTG
   SEQ ID NO: 57
803-1- Primer 2
   CAATTTGAGCTCTAACCAAATCTCTACCACACAACTTAATAAC
   SEQ ID NO: 58
803-1- Primer 3 CGGTTCTAGAAAGAGAGATTTGTACTCTGCTTTGG
   SEQ ID NO: 59
803-1- Primer 4 CCAAAGCAGAGTACAAATCTCTCTTTCTAGAACCG
   SEQ ID NO: 60

Relaxin 800803 carrier was used as a template of PCR site-directed mutagenesis, mutation process was the same as that described in Example 2. DNA Sequence of 800803-1 precursor is as follows:

GATTCTTGGATGGAAGAAGTTATTAAGTTGTGTGGTAGAGATTTGGTTA
GAGCTCAAATTGCTATTTGTGGTATGTCTACTTGGTCTAAGAGAAAGCCAAC
TGGTTACGGTTCTAGAAAGAGAGATTTGTACTCTGCTTTGGCTAACAAGTGT
TGTCATGTTGGTTGTACTAAGAGATCTTTGGCTAGATTTTGTTAA
SEQ ID NO: 61

Amino acid sequence coded by DNA above is as follows:

DSWMEEVIKLCGRDLVRAQIAICGMSTWSKRKPTGYGSRKRDLYSALAN
KCCHVGCTKRSLARFC SEQ ID NO: 6.

### 2. Transformation, screening and inducible expression of the recombinant human relaxin 800803-1

Procedure for transformation, screening and inducible expression of the recombinant human relaxin 800803-1 was the same as that described in Example 1.

Sequences of resulting recombinant human relaxin analog 800803-1 were as follows:

| | |
|---|---|
| chain B (D^{B14}) DSWMEEVIKLCGRDLVRAQIAICGMSTWS | SEQ ID NO: 137 |
| chain A (D^{A1}) DLYSALANKCCHVGCTKRSLARFC | SEQ ID NO: 138. |

### Example 7 Cloning and expression of recombinant human relaxin 800805

### 1. Construction of recombinant human relaxin 800805 expression vector

Relaxin 800805 was synthesized by PCR site-directed mutagenesis. Two single-stranded DNA fragments synthesized by Invitrogen Company were used as site-directed mutagenesis primers, sequences were as follows:
805- Primer 1 
GTCTACTTGGTCTAAGAGAGGTGGTGGTCCAAGAAGACAATTGTACTCT
GCTTTG SEQ ID NO: 62
805- Primer 2

CAAAGCAGAGTACAATTGTCTTCTTGGACCACCACCTCTCTTAGACCAA
GTAGAC SEQ ID NO: 63

Relaxin 800801 carrier was used as a template of PCR site-directed mutagenesis, mutation process was the same as that described in Example 2. DNA Sequence of 800805 precursor is as follows:

GATTCTTGGATGGAAGAAGTTATTAAGTTGTGTGGTAGAGAATTGGTTA
GAGCTCAAATTGCTATTTGTGGTATGTCTACTTGGTCTAAGAGAGGTGGTGG
TCCAAGAAGACAATTGTACTCTGCTTTGGCTAACAAGTGTTGTCATGTTGGT
TGTACTAAGAGATCTTTGGCTAGATTTTGTTAA SEQ ID NO: 64

Amino acid sequence coded by DNA above is as follows:

DSWMEEVIKLCGRELVRAQIAICGMSTWSKRGGGPRRQLYSALANKCCH
VGCTKRSLARFC SEQ ID NO: 7

### 2. Transformation, screening and inducible expression of the recombinant human relaxin 800805

Procedure for transformation, screening and inducible expression of the recombinant human relaxin 800805 was the same as that described in Example 1.

Sequences of resulting recombinant human relaxin 800805 (WT, wild-type) were as follows:

| | |
|---|---|
| chain B (WT) DSWMEEVIKLCGRELVRAQIAICGMSTWS | SEQ ID NO: 134 |
| chain A (WT) QLYSALANKCCHVGCTKRSLARFC | SEQ ID NO: 135. |

### Example 8 Cloning and expression of recombinant human relaxin 800805-1

### 1. Construction of recombinant human relaxin 800805-1 expression vector

Relaxin 800805-1 was synthesized by PCR site-directed mutagenesis. Four single-stranded DNA fragments synthesized by Invitrogen Company were used as site-directed mutagenesis primers, sequences were as follows:
805-1- Primer 1
   GTTATTAAGTTGTGTGGTAGAGATTTGGTTAGAGCTCAAATTG
   SEQ ID NO: 65
805-1- Primer 2
   CAATTTGAGCTCTAACCAAATCTCTACCACACAACTTAATAAC
   SEQ ID NO: 66
805-1- Primer 3 GGTGGTCCAAGAAGAGATTTGTACTCTGCTTTGG
   SEQ ID NO: 67
805-1- Primer 4 CCAAAGCAGAGTACAAATCTCTTCTTGGACCACC
   SEQ ID NO: 68

Relaxin 800805 carrier was used as a template of PCR site-directed mutagenesis, mutation process was the same as that described in Example 2. DNA Sequence of 800805-1 precursor is as follows:

GATTCTTGGATGGAAGAAGTTATTAAGTTGTGTGGTAGAGATTTGGTTA
GAGCTCAAATTGCTATTTGTGGTATGTCTACTTGGTCTAAGAGAGGTGGTGG
TCCAAGAAGAGATTTGTACTCTGCTTTGGCTAACAAGTGTTGTCATGTTGGT
TGTACTAAGAGATCTTTGGCTAGATTTTGTTAA SEQ ID NO: 69

Amino acid sequence coded by DNA above is as follows:

DSWMEEVIKLCGRDLVRAQIAICGMSTWSKRGGGPRRDLYSALANKCCH
VGCTKRSLARFC SEQ ID NO: 8.

### 2. Transformation, screening and inducible expression of the recombinant human relaxin 800805-1

Procedure for transformation, screening and inducible expression of the recombinant human relaxin 800805-1 was the same as that described in Example 1.

Sequences of resulting recombinant human relaxin analog 800805-1 were as follows:

| | |
|---|---|
| chain B (D^{B14}) DSWMEEVIKLCGRDLVRAQIAICGMSTWS | SEQ ID NO: 137 |

| | |
|---|---|
| chain A (D^{A1}) DLYSALANKCCHVGCTKRSLARFC | SEQ ID NO: 138. |

### Example 9 Cloning and expression of recombinant human relaxin 800806

### 1. Construction of recombinant human relaxin 800806 expression vector

Relaxin 800806 was synthesized by PCR site-directed mutagenesis. Two single-stranded DNA fragments synthesized by Invitrogen Company were used as site-directed mutagenesis primers, sequences were as follows:
806- Primer 1 
GTCTACTTGGTCTAAGAGAGGTGGTGGTCCAAAGAGACAATTGTACTCT
GCT
SEQ ID NO: 70
806- Primer 2

AGCAGAGTACAATTGTCTCTTTGGACCACCACCTCTCTTAGACCAAGTA
GAC
SEQ ID NO: 71

Relaxin 800801 carrier was used as a template of PCR site-directed mutagenesis, mutation process was the same as that described in Example 2. DNA Sequence of 800806 precursor is as follows:

GATTCTTGGATGGAAGAAGTTATTAAGTTGTGTGGTAGAGAATTGGTTA
GAGCTCAAATTGCTATTTGTGGTATGTCTACTTGGTCTAAGAGAGGTGGTGG
TCCAAAGAGACAATTGTACTCTGCTTTGGCTAACAAGTGTTGTCATGTTGGT
TGTACTAAGAGATCTTTGGCTAGATTTTGTTAA SEQ ID NO: 72

Amino acid sequence coded by DNA above is as follows:

DSWMEEVIKLCGRELVRAQIAICGMSTWSKRGGGPKRQLYSALANKCCH
VGCTKRSLARFC SEQ ID NO: 9.

### 2. Transformation, screening and inducible expression of the recombinant human relaxin 800806

Procedure for transformation, screening and inducible expression of the recombinant human relaxin 800806 was the same as that described in Example 1.

Sequences of resulting recombinant human relaxin 800806 (WT, wild-type) were as follows:

| | |
|---|---|
| chain B (WT) DSWMEEVIKLCGRELVRAQIAICGMSTWS | SEQ ID NO: 134 |
| chain A (WT) QLYSALANKCCHVGCTKRSLARFC | SEQ ID NO: 135. |

### Example 10 Cloning and expression of recombinant human relaxin 800806-1

### 1. Construction of recombinant human relaxin 800806-1 expression vector

Relaxin 800806-1 was synthesized by PCR site-directed mutagenesis. Four single-stranded DNA fragments synthesized by Invitrogen Company were used as site-directed mutagenesis primers, sequences were as follows:
806-1- Primer 1
   GTTATTAAGTTGTGTGGTAGAGATTTGGTTAGAGCTCAAATTG
   SEQ ID NO: 73
806-1- Primer 2
   CAATTTGAGCTCTAACCAAATCTCTACCACACAACTTAATAAC
   SEQ ID NO: 74
806-1- Primer 3 GGTGGTGGTCCAAAGAGAGATTTGTACTCTGCTTTGG
   SEQ ID NO: 75
806-1- Primer 4 CCAAAGCAGAGTACAAATCTCTCTTTGGACCACCACC
   SEQ ID NO: 76

Relaxin 800806 carrier was used as a template of PCR site-directed mutagenesis, mutation process was the same as that described in Example 2. DNA Sequence of 800806-1 precursor is as follows:

GATTCTTGGATGGAAGAAGTTATTAAGTTGTGTGGTAGAGATTTGGTTA
GAGCTCAAATTGCTATTTGTGGTATGTCTACTTGGTCTAAGAGAGGTGGTGG
TCCAAAGAGAGATTTGTACTCTGCTTTGGCTAACAAGTGTTGTCATGTTGGT
TGTACTAAGAGATCTTTGGCTAGATTTTGTTAA SEQ ID NO: 77

Amino acid sequence coded by DNA above is as follows:

DSWMEEVIKLCGRDLVRAQIAICGMSTWSKRGGGPKRDLYSALANKCCH
VGCTKRSLARFC SEQ ID NO: 10

### 2. Transformation, screening and inducible expression of the recombinant human relaxin 800806-1

Procedure for transformation, screening and inducible expression of the recombinant human relaxin 800806-1 was the same as that described in Example 1.

Sequences of resulting recombinant human relaxin analog 800806-1 were as follows:

| | |
|---|---|
| chain B (D^{B14}) DSWMEEVIKLCGRDLVRAQIAICGMSTWS | SEQ ID NO: 137 |
| chain A (D^{A1}) DLYSALANKCCHVGCTKRSLARFC | SEQ ID NO: 138. |

### Example 11 Cloning and expression of recombinant human relaxin 800808

### 1. Construction of recombinant human relaxin 800808 expression vector

Relaxin 800808 was synthesized by PCR site-directed mutagenesis. Two single-stranded DNA fragments synthesized by Invitrogen Company were used as site-directed mutagenesis primers, sequences were as follows:
808- Primer 1 
GGGTATCTCTCGAGAAAAGAGAAGAAGGTGAACCAAAGGATTCTTGGA
TGGAAGAAG SEQ ID NO: 78
808- Primer 2

CTTCTTCCATCCAAGAATCCTTTGGTTCACCTTCTTCTCTTTTCTCGAGA
GATACCC SEQ ID NO: 79

Relaxin 800800 carrier was used as a template of PCR site-directed mutagenesis, mutation process was the same as that described in Example 2. DNA Sequence of 800808 precursor is as follows:

GAAGAAGGTGAACCAAAGGATTCTTGGATGGAAGAAGTTATTAAGTTG
TGTGGTAGAGAATTGGTTAGAGCTCAAATTGCTATTTGTGGTATGTCTACTT
GGTCTAAGAGACAATTGTACTCTGCTTTGGCTAACAAGTGTTGTCATGTTGG
TTGTACTAAGAGATCTTTGGCTAGATTTTGTTAA SEQ ID NO: 80

Amino acid sequence coded by DNA above is as follows:

EEGEPKDSWMEEVIKLCGRELVRAQIAICGMSTWSKRQLYSALANKCCHV
GCTKRSLARFC SEQ ID NO: 11

### 2. Transformation, screening and inducible expression of the recombinant human relaxin 800808

Procedure for transformation, screening and inducible expression of the recombinant human relaxin 800808 was the same as that described in Example 1.

Sequences of resulting recombinant human relaxin 800808 (WT, wild-type) were as follows:

| | |
|---|---|
| chain B (WT) DSWMEEVIKLCGRELVRAQIAICGMSTWS | SEQ ID NO: 134 |
| chain A (WT) QLYSALANKCCHVGCTKRSLARFC | SEQ ID NO: 135. |

### Example 12 Cloning and expression of recombinant human relaxin 800808-1

### 1. Construction of recombinant human relaxin 800808-1 expression vector

Relaxin 800808-1 was synthesized by PCR site-directed mutagenesis. Four single-stranded DNA fragments synthesized by Invitrogen Company were used as site-directed mutagenesis primers, sequences were as follows:
808-1- Primer 1
   GTTATTAAGTTGTGTGGTAGAGATTTGGTTAGAGCTCAAATTG
   SEQ ID NO: 81
808-1- Primer 2
   CAATTTGAGCTCTAACCAAATCTCTACCACACAACTTAATAAC
   SEQ ID NO: 82
808-1- prime 3
   CTACTTGGTCTAAGAGAGATTTGTACTCTGCTTTGG
   SEQ ID NO: 83
808-1- Primer 4
   CCAAAGCAGAGTACAAATCTCTCTTAGACCAAGTAG
   SEQ ID NO: 84

Relaxin 800808 carrier was used as a template of PCR site-directed mutagenesis, mutation process was the same as that described in Example 2. DNA Sequence of 800808-1 precursor is as follows:

GAAGAAGGTGAACCAAAGGATTCTTGGATGGAAGAAGTTATTAAGTTG
TGTGGTAGAGATTTGGTTAGAGCTCAAATTGCTATTTGTGGTATGTCTACTT
GGTCTAAGAGAGATTTGTACTCTGCTTTGGCTAACAAGTGTTGTCATGTTGG
TTGTACTAAGAGATCTTTGGCTAGATTTTGTTAA SEQ ID NO: 85

Amino acid sequence coded by DNA above is as follows:

EEGEPKDSWMEEVIKLCGRDLVRAQIAICGMSTWSKRDLYSALANKCCHV
GCTKRSLARFC SEQ ID NO: 12

### 2. Transformation, screening and inducible expression of the recombinant human relaxin 800808-1

Procedure for transformation, screening and inducible expression of the recombinant human relaxin 800808-1 was the same as that described in Example 1.

Sequences of resulting recombinant human relaxin analog 800808-1 were as follows:

| | |
|---|---|
| chain B (D^{B14}) DSWMEEVIKLCGRDLVRAQIAICGMSTWS | SEQ ID NO: 137 |
| chain A (D^{A1}) DLYSALANKCCHVGCTKRSLARFC | SEQ ID NO: 138. |

### Example 13 Cloning and expression of recombinant human relaxin 800809

### 1. Construction of recombinant human relaxin 800809 expression vector

Relaxin 800809 was synthesized by PCR site-directed mutagenesis. Two single-stranded DNA fragments synthesized by Invitrogen Company were used as site-directed mutagenesis primers, sequences were as follows:
809- Primer 1 
GGGTATCTCTCGAGAAAAGAGAAGAAGGTGAACCAAAGAGAGATTCTT
GGATGGAAGAAG SEQ ID NO: 86
809- Primer 2

CTTCTTCCATCCAAGAATCTCTCTTTGGTTCACCTTCTTCTCTTTTCTCGA
GAGATACCC SEQ ID NO: 87

Relaxin 800800 carrier was used as a template of PCR site-directed mutagenesis, mutation process was the same as that described in Example 2. DNA Sequence of 800809 precursor is as follows:

GAAGAAGGTGAACCAAAGAGAGATTCTTGGATGGAAGAAGTTATTAAG
TTGTGTGGTAGAGAATTGGTTAGAGCTCAAATTGCTATTTGTGGTATGTCTA
CTTGGTCTAAGAGACAATTGTACTCTGCTTTGGCTAACAAGTGTTGTCATGT
TGGTTGTACTAAGAGATCTTTGGCTAGATTTTGTTAA SEQ ID NO: 88

Amino acid sequence coded by DNA above is as follows:

EEGEPKRDSWMEEVIKLCGRELVRAQIAICGMSTWSKRQLYSALANKCCH
VGCTKRSLARFC SEQ ID NO: 13.

### 2. Transformation, screening and inducible expression of the recombinant human relaxin 800809

Procedure for transformation, screening and inducible expression of the recombinant human relaxin 800809 was the same as that described in Example 1.

Sequences of resulting recombinant human relaxin 800809 (WT, wild-type) were as follows:

| | |
|---|---|
| chain B (WT) DSWMEEVIKLCGRELVRAQIAICGMSTWS | SEQ ID NO: 134 |
| chain A (WT) QLYSALANKCCHVGCTKRSLARFC | SEQ ID NO: 135. |

### Example 14 Cloning and expression of recombinant human relaxin 800809-1

### 1. Construction of recombinant human relaxin 800809-1 expression vector

Relaxin 800809-1 was synthesized by PCR site-directed mutagenesis. Four single-stranded DNA fragments synthesized by Invitrogen Company were used as site-directed mutagenesis primers, sequences were the same as 800808-1:
809-1- Primer 1
   GTTATTAAGTTGTGTGGTAGAGATTTGGTTAGAGCTCAAATTG
   SEQ ID NO: 89
809-1- Primer 2
   CAATTTGAGCTCTAACCAAATCTCTACCACACAACTTAATAAC
   SEQ ID NO: 90
809-1- Primer 3 CTACTTGGTCTAAGAGAGATTTGTACTCTGCTTTGG
   SEQ ID NO: 91
809-1- Primer 4 CCAAAGCAGAGTACAAATCTCTCTTAGACCAAGTAG
   SEQ ID NO: 92

Relaxin 800809 carrier was used as a template of PCR site-directed mutagenesis, mutation process was the same as that described in Example 2. DNA Sequence of 800809-1 precursor is as follows:

GAAGAAGGTGAACCAAAGAGAGATTCTTGGATGGAAGAAGTTATTAAG
TTGTGTGGTAGAGATTTGGTTAGAGCTCAAATTGCTATTTGTGGTATGTCTA
CTTGGTCTAAGAGAGATTTGTACTCTGCTTTGGCTAACAAGTGTTGTCATGT
TGGTTGTACTAAGAGATCTTTGGCTAGATTTTGTTAA SEQ ID NO: 93

Amino acid sequence coded by DNA above is as follows:

EEGEPKRDSWMEEVIKLCGRDLVRAQIAICGMSTWSKRDLYSALANKCCH
VGCTKRSLARFC SEQ ID NO: 14.

### 2. Transformation, screening and inducible expression of the recombinant human relaxin 800809-1

Procedure for transformation, screening and inducible expression of the recombinant human relaxin 800809-1 was the same as that described in Example 1.

Sequences of resulting recombinant human relaxin analog 800809-1 were as follows:

| | |
|---|---|
| chain B (D^{B14}) DSWMEEVIKLCGRDLVRAQIAICGMSTWS | SEQ ID NO: 137 |
| chain A (D^{A1}) DLYSALANKCCHVGCTKRSLARFC | SEQ ID NO: 138. |

### Example 15 Cloning and expression of recombinant human relaxin 800810

### 1. Construction of recombinant human relaxin 800810 expression vector

Relaxin 800810 was synthesized by PCR site-directed mutagenesis. Two single-stranded DNA fragments synthesized by Invitrogen Company were used as site-directed mutagenesis primers, sequences were as follows:
810- Primer 1
   GTTATTAAGTTGTGTGGTAGAGATTTGGTTAGAGCTCAAATTG
   SEQ ID NO: 94
810- Primer 2
   CAATTTGAGCTCTAACCAAATCTCTACCACACAACTTAATAAC
   SEQ ID NO: 95

Relaxin 800800 carrier was used as a template of PCR site-directed mutagenesis, mutation process was the same as that described in Example 2. DNA Sequence of 800810 precursor is as follows:

GATTCTTGGATGGAAGAAGTTATTAAGTTGTGTGGTAGAGATTTGGTTA
GAGCTCAAATTGCTATTTGTGGTATGTCTACTTGGTCTAAGAGACAATTGTA
CTCTGCTTTGGCTAACAAGTGTTGTCATGTTGGTTGTACTAAGAGATCTTTG
GCTAGATTTTGTTAA SEQ ID NO: 96

Amino acid sequence coded by DNA above is as follows:

DSWMEEVIKLCGRDLVRAQIAICGMSTWSKRQLYSALANKCCHVGCTKR
SLARFC SEQ ID NO: 15.

### 2. Transformation, screening and inducible expression of the recombinant human relaxin 800810

Procedure for transformation, screening and inducible expression of the recombinant human relaxin 800810 was the same as that described in Example 1.

Sequences of resulting recombinant human relaxin 800810 were as follows:

| | |
|---|---|
| chain B (D^{B14}) DSWMEEVIKLCGRDLVRAQIAICGMSTWS | SEQ ID NO: 137 |
| chain A (WT) QLYSALANKCCHVGCTKRSLARFC | SEQ ID NO: 135. |

### Example 16 Cloning and expression of recombinant human relaxin 800810-1

### 1. Construction of recombinant human retaxin 800810-1 expression vector

Relaxin 800810-1 was synthesized by PCR site-directed mutagenesis. Two single-stranded DNA fragments synthesized by Invitrogen Company were used as site-directed mutagenesis primers, sequences were as follows:
810-1- Primer 1
   CTACTTGGTCTAAGAGAGATTTGTACTCTGCTTTGG SEQ ID NO: 97
810-1- Primer 2
   CCAAAGCAGAGTACAAATCTCTCTTAGACCAAGTAG SEQ ID NO: 98

Relaxin 800810 carrier was used as a template of PCR site-directed mutagenesis, mutation process was the same as that described in Example 2. DNA Sequence of 800810-1 precursor is as follows:

GATTCTTGGATGGAAGAAGTTATTAAGTTGTGTGGTAGAGATTTGGTTA
GAGCTCAAATTGCTATTTGTGGTATGTCTACTTGGTCTAAGAGAGATTTGTA
CTCTGCTTTGGCTAACAAGTGTTGTCATGTTGGTTGTACTAAGAGATCTTTG
GCTAGATTTTGTTAA SEQ ID NO: 99

Amino acid sequence coded by DNA above is as follows:

DSWMEEVIKLCGRDLVRAQIAICGMSTWSKRDLYSALANKCCHVGCTKR
SLARFC SEQ ID NO: 16.

### 2. Transformation, screening and inducible expression of the recombinant human relaxin 8008 10-1

Procedure for transformation, screening and inducible expression of the recombinant human relaxin 800810-1 was the same as that described in Example 1.

Sequences of resulting recombinant human relaxin analog 800810-1 were as follows:

| | |
|---|---|
| chain B (D^{B14}) DSWMEEVIKLCGRDLVRAQIAICGMSTWS | SEQ ID NO: 137 |
| chain A (D^{A1}) DLYSALANKCCHVGCTKRSLARFC | SEQ ID NO: 138. |

### Example 17 Cloning and expression of recombinant human relaxin 800811

### 1. Construction of recombinant human relaxin 800811 expression vector

Relaxin 800811 was synthesized by PCR site-directed mutagenesis. Two single-stranded DNA fragments synthesized by Invitrogen Company were used as site-directed mutagenesis primers, sequences were as follows:
811- Primer 1
   GTTATTAAGTTGTGTGGTAGAGATTTGGTTAGAGCTCAAATTG
   SEQ ID NO: 100
811- Primer 2
   CAATTTGAGCTCTAACCAAATCTCTACCACACAACTTAATAAC
   SEQ ID NO: 101

Relaxin 800804 carrier was used as a template of PCR site-directed mutagenesis, mutation process was the same as that described in Example 2. DNA Sequence of 800811 precursor is as follows:

GATTCTTGGATGGAAGAAGTTATTAAGTTGTGTGGTAGAGATTTGGTTA
GAGCTCAAATTGCTATTTGTGGTATGTCTACTTGGTCTAAGAGAAAGCCAAC
TGGTTACGGTTCTAAGAGACAATTGTACTCTGCTTTGGCTAACAAGTGTTGT
CATGTTGGTTGTACTAAGAGATCTTTGGCTAGATTTTGTTAA
SEQ ID NO: 102

Amino acid sequence coded by DNA above is as follows:

DSWMEEVIKLCGRDLVRAQIAICGMSTWSKRKPTGYGSKRQLYSALANK

CCHVGCTKRSLARFC SEQ ID NO: 17.

### 2. Transformation, screening and inducible expression of the recombinant human relaxin 800811

Procedure for transformation, screening and inducible expression of the recombinant human retaxin 800811 was the same as that described in Example 1.

Sequences of resulting recombinant human relaxin analog 800811 were as follows:

| | |
|---|---|
| chain B (D^{B14}) DSWMEEVIKLCGRDLVRAQIAICGMSTWS | SEQ ID NO: 137 |
| chain A (WT) QLYSALANKCCHVGCTKRSLARFC | SEQ ID NO: 135. |

### Example 18 Cloning and expression of recombinant human relaxin 800813

### 1. Construction of recombinant human relaxin 800813 expression vector

Relaxin 800813 3 was synthesized by PCR site-directed mutagenesis. Two single-stranded DNA fragments synthesized by Invitrogen Company were used as site-directed mutagenesis primers, sequences were as follows:
813- Primer 1
   CTGGTTACGGTTCTAAGAGAGAATTGTACTCTGCTTTGGC
   SEQ ID NO: 103
813- Primer 2 GCCAAAGCAGAGTACAATTCTCTCTTAGAACCGTAAGCAG SEQ ID NO: 104

Relaxin 800811 carrier was used as a template of PCR site-directed mutagenesis, mutation process was the same as that described in Example 2. DNA Sequence of 800813 precursor is as follows:

GATTCTTGGATGGAAGAAGTTATTAAGTTGTGTGGTAGAGATTTGGTTA
GAGCTCAAATTGCTATTTGTGGTATGTCTACTTGGTCTAAGAGAAAGCCAAC
TGGTTACGGTTCTAAGAGAGAATTGTACTCTGCTTTGGCTAACAAGTGTTGT
CATGTTGGTTGTACTAAGAGATCTTTGGCTAGATTTTGTTAA
SEQ ID NO: 105

Amino acid sequence coded by DNA above is as follows:

DSWMEEVIKLCGRDLVRAQIAICGMSTWSKRKPTGYGSKRELYSALANKC
CHVGCTKRSLARFC SEQ ID NO: 18.

### 2. Transformation, screening and inducible expression of the recombinant human relaxin 800813

Procedure for transformation, screening and inducible expression of the recombinant human relaxin 800813 was the same as that described in Example 1.

Sequences of resulting recombinant human relaxin analog 800813 were as follows:

| | |
|---|---|
| chain B (D^{B14}) DSWMEEVIKLCGRDLVRAQIAICGMSTWS | SEQ ID NO: 137 |
| chain A (E^{A1}) ELYSALANKCCHVGCTKRSLARFC | SEQ ID NO: 136. |

### Example 19 Cloning and expression of recombinant human relaxin 800814

### 1. Construction of recombinant human relaxin 800814 expression vector

Relaxin 800814 was synthesized by PCR site-directed mutagenesis. Two single-stranded DNA fragments synthesized by Invitrogen Company were used as site-directed mutagenesis primers, sequences were as follows:
814- Primer 1 CTGGTTACGGTTCTAAGAGAGATTTGTACTCTGCTTTGGC SEQ ID NO: 106
814- Primer 2
   GCCAAAGCAGAGTACAAATCTCTCTTAGAACCGTAACCAG
   SEQ ID NO: 107

Relaxin 800811 carrier was used as a template of PCR site-directed mutagenesis, mutation process was the same as that described in Example 2. DNA Sequence of 800814 precursor is as follows:

GATTCTTGGATGGAAGAAGTTATTAAGTTGTGTGGTAGAGATTTGGTTA
GAGCTCAAATTGCTATTTGTGGTATGTCTACTTGGTCTAAGAGAAAGCCAAC
TGGTTACGGTTCTAAGAGAGATTTGTACTCTGCTTTGGCTAACAAGTGTTGT
CATGTTGGTTGTACTAAGAGATCTTTGGCTAGATTTTGTTAA
SEQ ID NO: 108

Amino acid sequence coded by DNA above is as follows:

DSWMEEVIKLCGRDLVRAQIAICGMSTWSKRKPTGYGSKRDLYSALANK
CCHVGCTKRSLARFC SEQ ID NO: 19.

### 2. Transformation, screening and inducible expression of the recombinant human relaacin 800814

Procedure for transformation, screening and inducible expression of the recombinant human relaxin 800814 was the same as that described in Example 1.

Sequences of resulting recombinant human relaxin analog 800814 were as follows:

| | |
|---|---|
| chain B (D^{B14}) DSWMEEVIKLCGRDLVRAQIAICGMSTWS | SEQ ID NO: 137 |
| chain A (D^{A1}) DLYSALANKCCHVGCTKRSLARFC | SEQ ID NO: 138. |

### Example 20 Cloning and expression of recombinant human relaxin 800816

### 1. Construction of recombinant human relaxin 800816 expression vector

Relaxin 800816 was synthesized by PCR site-directed mutagenesis. Two single-stranded DNA fragments synthesized by Invitrogen Company were used as site-directed mutagenesis primers, sequences were as follows:
816- Primer 1
   ATTAAGTTGTGTGGTAGAAACTTGGTTAGAGCTCAAATTGC
   SEQ ID NO: 109
816- Primer 2
   GCAATTTGAGCTCTAACCAAGTTTCTACCACACAACTTAAT
   SEQ ID NO: 110

Relaxin 800804 carrier was used as a template of PCR site-directed mutagenesis, mutation process was the same as that described in Example 2. DNA Sequence of 800816 precursor is as follows:

GATTCTTGGATGGAAGAAGTTATTAAGTTGTGTGGTAGAAACTTGGTTA
GAGCTCAAATTGCTATTTGTGGTATGTCTACTTGGTCTAAGAGAAAGCCAAC
TGGTTACGGTTCTAAGAGACAATTGTACTCTGCTTTGGCTAACAAGTGTTGT
CATGTTGGTTGTACTAAGAGATCTTTGGCTAGATTTTGTTAA
SEQ ID NO: 111

Amino acid sequence coded by DNA above is as follows:

DSWMEEVIKLCGRNLVRAQIAICGMSTWSKRKPTGYGSKRQLYSALANK
CCHVGCTKRSLARFC SEQ ID NO: 20.

### 2. Transformation, screening and inducible expression of the recombinant human relaxin 800816

Procedure for transformation, screening and inducible expression of the recombinant human relaxin 800816 was the same as that described in Example 1.

Sequences of resulting recombinant human relaxin analog 800816 were as follows:

| | |
|---|---|
| chain B (N^{D14}) DSWMEEVIKLCGRNLVRAQIAICGMSTWS | SEQ ID NO: 139 |
| chain A (WT) QLYSALANKCCHVGCTKRSLARFC | SEQ ID NO: 135, |

### Example 21 Cloning and expression of recombinant human relaxin 800847Y

### 1. Construction of recombinant human relaxin 800847Y expression vector

Relaxin 800847Y was synthesized by PCR site-directed mutagenesis. Two single-stranded DNA fragments synthesized by Invitrogen Company were used as site-directed mutagenesis primers, sequences were as follows:
847Y- Primer 1 AAGTTGTGTGGTAGAGAATTGGTTAGAGCTCAAA
   SEQ ID NO: 112
847Y- Primer 2 TTTGAGCTCTAACCAATTCTCTACCACACAACTT
   SEQ ID NO: 113

Relaxin 800814 carrier was used as a template of PCR site-directed mutagenesis, mutation process was the same as that described in Example 2. DNA Sequence of 800847Y precursor is as follows:

GATTCTTGGATGGAAGAAGTTATTAAGTTGTGTGGTAGAGAATTGGTTA
GAGCTCAAATTGCTATTTGTGGTATGTCTACTTGGTCTAAGAGAAAGCCAAC
TGGTTACGGTTCTAAGAGAGATTTGTACTCTGCTTTGGCTAACAAGTGTTGT
CATGTTGGTTGTACTAAGAGATCTTTGGCTAGATTTTGTTAA SEQ ID NO:
114

Amino acid sequence coded by DNA above is as follows:

DSWMEEVIKLCGRELVRAQIAICGMSTWSKRKPTGYGSKRDLYSALANKC
CHVGCTKRSLARFC SEQ ID NO: 21

### 2. Transformation, screening and inducible expression of the recombinant human relaxin 800847Y

Procedure for transformation, screening and inducible expression of the recombinant human relaxin 800847Y was the same as that described in Example 1.

Sequences of resulting recombinant human relaxin analog 800847Y were as follows:

| | |
|---|---|
| chain B (WT) DSWMEEVIKLCGRELVRAQIAICGMSTWS | SEQ ID NO: 134 |
| chain A (D^{A1}) DLYSALANKCCHVGCTKRSLARFC | SEQ ID NO: 138. |

### Example 22 Cloning and expression of recombinant human relaxin 800851Y

### 1. Construction of recombinant human relaxin 800851Y expression vector

Relaxin 800851 Y was synthesized by PCR site-directed mutagenesis. Two single-stranded DNA fragments synthesized by Invitrogen Company were used as site-directed mutagenesis primers, sequences were as follows:
851Y- Primer 1
   GGGGTATCTCTCGAGAAAAGATGGATGGAAGAAGTTATTAAG
   SEQ ID NO: 115
851Y- Primer 2
   CTTAATAACTTCTTCCATCCATCTTTTCTCGAGAGATACCCC
   SEQ ID NO: 116

Relaxin 800814 carrier was used as a template of PCR site-directed mutagenesis, mutation process was the same as that described in Example 2. DNA Sequence of 800851 Y precursor is as follows:

TGGATGGAAGAAGTTATTAAGTTGTGTGGTAGAGATTTGGTTAGAGCTC
AAATTGCTATTTGTGGTATGTCTACTTGGTCTAAGAGAAAGCCAACTGGTTA
CGGTTCTAAGAGAGATTTGTACTCTGCTTTGGCTAACAAGTGTTGTCATGTT
GGTTGTACTAAGAGATCTTTGGCTAGATTTTGTTAA SEQ ID NO: 117

Amino acid sequence coded by DNA above is as follows:

WMEEVIKLCGRDLVRAQIAICGMSTWSKRKPTGYGSKRDLYSALANKCC
HVGCTKRSLARFC SEQ ID NO: 22.

### 2. Transformation, screening and inducible expression of the recombinant human relaxin 800851 Y

Procedure for transformation, screening and inducible expression of the recombinant human relaxin 800851 Y was the same as that described in Example 1.

Sequences of resulting recombinant human relaxin analog 800851Y were as follows:

| | |
|---|---|
| chain B (D^{B14} DSWMEEVIKLCGRDLVRAQIAICGMSTWS | SEQ ID NO: 137 |
| chain A (D^{A1}) DLYSALANKCCHVGCTKRSLARFC | SEQ ID NO: 138. |

### Example 23 Cloning and expression of recombinant human relaxin 800828

### 1. Construction of recombinant human relaxin 800828 expression vector

The full length DNA sequence of codon-optimized human relaxin was synthesized by Overlap PCR method via introducing Ndel cleavage site (CATATG) into 5' terminal and BamHI cleavage site (GGATCC) into 3' terminal. Six single-stranded DNA fragments synthesized by Invitrogen Company were used as synthetic primers, sequences are as follows:
828- Primer 1 
CATATGAAGAAAAACATCGCGTTCCTGCTGAAACGTGACTCTTGGAT
GGA SEQ ID NO: 118
828-Primer 2 
GCACGAACCAGTTCACGACCGCACAGTTTGATAACTTCTTCCATCC
AAGAGTCACGTTT SEQ ID NO: 119
828- Primer 3 
CGTGAACTGGTTCGTGCGCAAATTGCGATCTGCGGTATGTCTACCT
GGTCTAAACGTAA SEQ ID NO: 120
828-Primer 4 
CAGCTGACGTTTTTACGAGAACCGTAACCGGTCGGTTTACGTTTA
GACCAGGTAGACA SEQ ID NO: 121
828- Primer 5 
CTCGTAAAAACGTCAGCTGTACTCTGCGCTGGCGAACAAATGCTG
CCACGTTGGTTGC SEQ ID NO: 122
828- Primer 6

GGATCCTTAGCAGAAACGCGCCAGAGAACGTTTGGTGCAACCAAC
GTGGCAG SEQ ID NO: 123

Relaxin was synthesized by using KOD plus kit (TOYOBO, Cat. KOD-201), and the reaction was performed by two-step PCR. PCR step 1, 25µL reaction system: 2.5µL of 10×KOD buffer, 2.5µL of 2mM dNTPs, primers 1, 2, 3, 4, 5, 6 (10µM) each 1µL, 0.5µL of KOD plus, 1µL of 25mM MgSO4, 12.5 µL of ddH₂O. Reaction program: 94°C 5 minutes; 94 °C 30 seconds, 60 °C 30 seconds, 68°C 30 seconds, for 30 cycles of amplification; then 68 °C 30 minutes to terminate PCR amplification process. PCR step 2, 25µL reaction system: 2.5µL of 10×KOD buffer, 2.5µL of 2mM dNTPs, primers 1 and 6 (10µM) each 1µL, 1µL of round 1 PCR product, 0.5µL of KOD plus, 1µL. of 25mM MgSO4, 15.5µL of ddH₂O. Reaction program: 94 °C 5 minutes; 94 °C 30 seconds, 68°C 60 seconds, for 30 cycles of amplification; then 68 °C 10 minutes to terminate PCR amplification process.

The synthesized DNA sequence and pET9a vector (Novagen, Cat. 69431-3) were respectively digested using Ndel/BamHI (Takara, Cat. D1161A/D1010A), obtained fragments were recovered by 1.2% agarose gel, linked by T4 DNA ligase (New England Biolabs, Cat. M0202V), and transformed into DH5α competent cells (Tiangen, Cat. CB101-02). Positive clones were picked, and sequenced by Invitrogen. DNA sequence of 800828 precursor is as follows:

CATATGAAGAAAAACATCGCGTTCCTGCTGAAACGTGACTCTTGGATG
GAAGAAGTTATCAAACTGTGCGGTCGTGAACTGGTTCGTGCGCAAATTGCG
ATCTGCGGTATGTCTACCTGGTCTAAACGTAAACCGACCGGTTACGGTTCTC
GTAAAAAACGTCAGCTGTACTCTGCGCTGGCGAACAAATGCTGCCACGTTG
GTTGCACCAAACGTTCTCTGGCGCGTTTCTGCTAAGGATCC
SEQ ID NO: 124

The underlined indicates cleavage sites.

Amino acid sequence coded by DNA above is as follows:

MKKNIAFLLKRDSWMEEVIKLCGRELVRAQIAICGMSTWSKRKPTGYGSR
KKRQLYSALANKCCHVGCTKRSLARFC SEQ ID NO: 23.

### 2. Inducible expression of small amount of recombinant human relaxin 800828

PET9a-relaxin 800828 plasmid with correct sequence was transformed into BL21 (DE3) competent cells (Tiangen, Cat. CB 105-02), monoclonal strain was picked for IPTG induction. Specific method was as follows: monoclonal strain was picked from a fresh plate, inoculated into LB medium which contained 10ml of ampicillin, cultured with shaking at 37°C until OD600 value reached 0.6. Portion of the sample was collected as a non-induced contrast and cryopreserved. The remaining sample was diluted to a final concentration of 1mM with addition of 1M of IPTG stock solution and incubated for additional 4 hours. The strain was harvested by centrifugation and analyzed in SDS-PAGE electrophoresis analysis after the induction.

Sequences of resulting recombinant human relaxin 800828 (WT, wild-type) were as follows:

| | |
|---|---|
| chain B (WT) DSWMEEVIKLCGRELVRAQIAICGMSTWS | SEQ ID NO: 134 |
| chain A (WT) QLYSALANKCCHVGCTKRSLARFC | SEQ ID NO: 135. |

### Example 24 Cloning and expression of recombinant human relaxin 800843

### 1. Construction of recombinant human relaxin 800843 expression vector

Relaxin 800843 was synthesized by PCR site-directed mutagenesis. Two single-stranded DNA fragments synthesized by Invitrogen Company were used as site-directed mutagenesis primers, sequences were as follows:
843- Primer 1 GTTCTCGTAAAAAACGTTGGCTGTACTCTGCGCTG
   SEQ ID NO: 125
843- Primer 2 CAGCGCAGAGTACAGCCAACGTTTTTTACGAGAAC
   SEQ ID NO: 126

Relaxin 800828 carrier was used as a template of PCR site-directed mutagenesis, mutation process was the same as that described in Example 18. DNA Sequence of 800843 precursor is as follows:

ATGAAGAAAAACATCGCGTTCCTGCTGAAACGTGACTCTTGGATGGAA
GAAGTTATCAAACTGTGCGGTCGTGAACTGGTTCGTGCGCAAATTGCGATCT
GCGGTATGTCTACCTGGTCTAAACGTAAACCGACCGGTTACGGTTCTCGTAA
AAAACGTTGGCTGTACTCTGCGCTGGCGAACAAATGCTGCCACGTTGGTTGC
ACCAAACGTTCTCTGGCGCGTTTCTGCTAA SEQ ID NO: 127

Amino acid sequence coded by DNA above is as follows:

MKKNIAFLLKRDSWMEEVIKLCGRELVRAQIAICGMSTWSKRKPTGYGSR
KKRWLYSALANKCCHVGCTKRSLARFC SEQ ID NO: 24.

### 2. Inducible expression of small amount of recombinant human relaxin 800843

Procedure for inducible expression of small amount of recombinant human relaxin 800843 was the same as that described in Example 16.

Sequences of resulting recombinant human relaxin analog 800843 were as follows:

| | |
|---|---|
| chain B (WT) DSWMEEVIKLCGRE-LVRAQIAICGMSTWS | SEQ ID NO: 134 |
| chain A (W^{A1}) WLYSALANKCCHVGCTKRSLARFC | SEQ ID NO: 141. |

### Example 25 Cloning and expression of recombinant human relaxin 800847

### 1. Construction of recombinant human relaxin 800847 expression vector

Relaxin 800847 was synthesized by PCR site-directed mutagenesis. Two single-stranded DNA fragments synthesized by Invitrogen Company were used as site-directed mutagenesis primers, sequences were as follows:
847- Primer 1 CAAACTGTGCGGTCGTGAACTGGTTCGTGCGCAAA
   SEQ ID NO: 128
847- Primer 2 TTTGCGCACGAACCAGTTCACGACCGCACAGTTTG
   SEQ ID NO: 129

Relaxin 800845 carrier was used as a template of PCR site-directed mutagenesis, mutation process was the same as that described in Example 18. DNA Sequence of 800847 precursor is as follows:

ATGAAGAAAAACATCGCGTTCCTGCTGAAACGTGACTCTTGGATGGAA
GAAGTTATCAAACTGTGCGGTCGTGAACTGGTTCGTGCGCAAATTGCGATCT
GCGGTATGTCTACCTGGTCTAAACGTAAACCGACCGGTTACGGTTCTAAACG
TGACCTGTACTCTGCGCTGGCGAACAAATGCTGCCACGTTGGTTGCACCAAA
CGTTCTCTGGCGCGTTTCTGCTAA SEQ ID NO: 130

Amino acid sequence coded by DNA above is as follows:

MKKNIAFLLKRDSWMEEVIKLCGRELVRAQIAICGMSTWSKRKPTGYGSK
RDLYSALANKCCHVGCTKRSLARFC SEQ ID NO: 25.

### 2. Inducible expression of small amount of recombinant human relaxin 800847

Procedure for inducible expression of small amount of recombinant human relaxin 800847 was the same as that described in Example 16.

Sequences of resulting recombinant human relaxin analog 800847 were as follows:

| | |
|---|---|
| chain B (WT) DSWMEEVIKLCGRELVRAQIAICGMSTWS | SEQ ID NO: 134 |
| chain A (O^{A1}) DLYSALANKCCHVGCTKRSLARFC | SEQ ID NO: 138. |

### Example 26 Cloning and expression of recombinant human relaxin 800851

### 1. Construction of recombinant human relaxin 800851 expression vector

Relaxin 800851 was synthesized by PCR site-directed mutagenesis. Two single-stranded DNA fragments synthesized by Invitrogen Company were used as site-directed mutagenesis primers, sequences were as follows:
851-Primer 1 CGCGTTCCTGCTGAAACGTTGGATGGAAGAAGTTATC
   SEQ ID N4: 131
851- Primer 2 GATAACTTCTTCCATCCAACGTTTCAGCAGGAACGCG
   SEQ ID NO: 132

Relaxin 800845 carrier was used as a template of PCR site-directed mutagenesis, mutation process was the same as that described in Example 18. DNA Sequence of 800851 precursor is as follows:

ATGAAGAAAAACATCGCGTTCCTGCTGAAACGTTGGATGGAAGAAGTT
ATCAAACTGTGCGGTCGTGACCTGGTTCGTGCGCAAATTGCGATCTGCGGTA
TGTCTACCTGGTCTAAACGTAAACCGACCGGTTACGGTTCTAAACGTGACCT
GTACTCTGCGCTGGCGAACAAATGCTGCCACGTTGGTTGCACCAAACGTTCT
CTGGCGCGTTTCTGCTAA SEQ ID NO: 133

Amino acid sequence coded by DNA above is as follows:

MKKNIAFLLKRWMEEVIKLCGRDLVRAQIAICGMSTWSKRKPTGYGSKRD
LYSALANKCCHVGCTKRSLARFC SEQ ID NO: 26.

### 2. Inducible expression of small amount of recombinant human relaxin 800851

Procedure for inducible expression of small amount of recombinant human relaxin 800851 was the same as that described in Example 16.

Sequences of resulting recombinant human relaxin analog 800851 were as follows:

| | |
|---|---|
| chain B (Del^{B1B2},DB¹⁴) WMEEVIKLCGRDLVRAQIAICGMSTWS | SEQ ID NO: 140 |
| chain A (D^{A1}) DLYSALANKCCHVGCTKRSLARFC SEQ | ID NO: 138. |

### Example 27 Fermentation of recombinant human relaxin 800828

1. Strain seed culture
   1) Strain: 800828 expression strain
   2) Media:
      a) LB medium: Yeast extract 5g/L, typtone 10 g/L, NaCl 5 g/L, 121°C 30min, high temperature sterilized,
      b) kanamycin stock solution: 50mg/mL, 0.22µm filter sterilized
      c) Fermentation medium:
         Tryptone 20g/L, Yeast extract 10 g/L, NaCl 10 g/L, Na₂HPO₄·12H₂O 4 g/L, KH₂PO4 2 g/L, K₂HPO4 2 g/L, MgSO₄ 1 g/L, glycerol 10 g/L, defoamer 5mL, after dissolution, the mixture was loaded into 5L of fermenterfor sterilization at 121°C for 30min.
      d) Supplementary medium:
         Tryptone100g/L, Yeast extract 50 g/L, glycerol 500 g/L, 121°C sterilized for 30min.
2. Fermentation process control
   1) Seed Activation: Seed stored in glycerol tube at -80°C were thawed at room temperature, 500uL of microbial suspension in glycerol tube was inoculated sterilely in 50 mL of LB medium, followed by addition of 5µL of kanamycin stock solution, cultured for 7 hours at 37°C, 200 rpm.
   2) Fermenter uploading: 50mL of activated seed was inoculated sterilely in 3L of fermentation medium, followed by addition of 30mL of kanamycin. Temperature condition of culture: 37°C, air flow rate: 0.5vvm, pressure of fermenter: 0.04 to 0.05 mpa, DO: 30% and above, pH: about 7.0, maintained with ammonia.
   3) About 4 hours after fermentation, samples were taken from broth per hour for measuring the value of OD600, and induction began while OD₆₀₀≥40.
   4) Inducer: 4mL of IPTG (1mol/L, sterilized by passing through 0.22µm filter membrane before use) was added and the final concentration of IPTG was 1 mmol/L, temperature was kept at 37°C during the induction phase, other conditions remained unchanged, the fermentation broth was induced for 12 hours.
   5) The fermenter was discharged when the induction ended, broth was measured for OD600 then, and centrifugated at 5000 rpm for 20min. cells were collected, and stored in -80°C refrigerator.

### Example 28 Fermentation of recombinant human relaxin 800814

1. Strain: 800828 expression strain
   2. 5L of fermenter operation process:
   1) Seed Activation:
      1 mL of strain in glycerol mentioned above was inoculated in 1000 mL of BMGY medium, and cultured for 20 hours at 30 °C, 220r/min (rpm).
   2) Inoculation:
      Activated seed was inoculated sterilely in 3L of fermentation medium (H₃PO₄ 26.7ml/L, CaSO₄ 0.93g/L, K₂SO₄ 18.2g/L, MgSO₄.7H₂O 14.9g/L, KOH 4.13g/L, glycerol 40g/L), followed by addition of 0.4% sterile PTM1 solution, and fermentation began.
   3) Initial stage of culture:
      Strain entered into exponential growth phase after a period of adjustment (10 to 12hr). DO of >30% required by cell growth was satisfied by increasing agitation speed and aeration rate. Rotate speed was increased by 50-100 rpm every time. Fermentation temperature was controlled at 37°C, tank pressure: 0.04 to 0.05Mpa, pH: 7.0.
   4) Glycerol supplemented stage:
      When substrate in the initial medium was consumed (18 to 24hr), 50% glycerol fed-batch was supplemented at a limited rate.
   5) Methanol induction phase:
      After 2 to 4h of cell growth in glycerol supplementation phase, glycerol supplementation was terminated, cells were starved for 30 minutes, so that glycerol can be completely exhausted, and then methanol was added for induction. After 70 to 96 hours, fermentation was ended, and the broth was centrifuged at 7000 rpm, the supernatant was collected.
   6) Yield of protein expression:
      One ml of fermentation supernatant was investigated for the protein content in the supernatant by using SDS-PAGE, correct expression was proved.

### Example 29 Purification and refolding of recombinant human relaxin 800828

1. Inclusion bodies purification
   *1) E. coli,* wet weight of 100g, was suspended in 300ml of 50mM Tris pH8.5 and 0.2% EDTA, and ultrasonicated. After centrifugation at 10000rpm 4°C for 1hr, supernatant was removed.
   2) Pellet was fully suspended in 300ml of 2M Urea, 50mM Tris pH8.5, 0.2% EDTA. After centrifugation, supernatant was removed. Repeat once.
   3) Pellet was fully suspended in 200ml of 8M Urea, 50mM Tris pH8.5, 0.2% EDTA. After centrifugation, supernatant was removed.
   4) Pellet was fully suspended in 200ml of 50mM Tris pH8.5, 0.2% EDTA. After centrifugation, supernatant was removed. The pellet was weighed.
   5) Pellet was fully suspended in 6 volumes of 6M GdnCl 50mM Tris pH8.5, 0.2% FDTA, 10mM DTT, dissolved at room temperature for 2hr. After centrifugation at 10000rpm 4°C for 1hr, the supernatant was collected.
   6) Various rinsed components were detected by electrophoresis.
2. Inclusion bodies refolding
   1) Obtained inclusion bodies were diluted in 2 volumes of 50mM Tris pH8.5, 0.2% EDTA, fully mixed and placed in the 4°C refrigerator overnight.
   2) Refolding process was detected by RP-UPLC test; after reaction, the products were purified by preparative liquid phase; and then molecular weight was detected by LC-MS.
      a) RP-UPLC Condition: Waters UPLC BioHClass, ACQUITY UPLC BEH 300 C4 (1.7µm 2.1 x 100mm), mobile phase: A solution of 0.1% TFA; B solution of ACN, flow rate at 0.3ml/min, linear gradient: 0.5min (10 B%) to 9min (60 B%).
      b) Preparation: Water AutoPurifer, Kromasil 10-100-C18, 30 x 250mm; mobile phase: A solution was 0.1% TFA; B solution ACN, flow rate at 40 ml/min, linear gradient of 2min (25% B) to 15min (50% B).
      c) LC-MS method: Finnigan LCQ Ad, Jupiter C4 (5u, 300A, 250 × 4,6mm), mobile phase: A solution of 0.2% FA; B solution of 0.18% FA/CAN, linear gradient: 0min (5% B) to 20min (50B%).
3. Digestion: 50mM Tris pH8.5, 5mM Ca2⁺, Trypsin 1:1300, CPB 1:50, substrate concentration of 1mg/ml, reaction temperature of 30 °C. The reaction process was detected by UPLC. After that, the reaction system was adjusted to pH 3-4 to terminate the reaction, and centrifuged to remove the pellet. Supernatant was filtered through a 0.22µm membrane and purified by preparative RP-HPLC. Reverse phase preparative gradient was 2min (20% B) to 15min (45% B), other test conditions were as above mentioned. The product (No. 828) was lyophilized and obtained. Purity was tested by RP-UPLC and IEC-HPLC.
   1) RP-UPLC conditions were as above mentioned,
   2) IEC-HPLC: Waters UPLC BioHClass, Protein-Pak Hi Res CM (7µm, 4.6 x 100mm), A solution: 20mM HEPES pH8.0; B solution: 0.5M NaCl 20mM HEPES pH8.0, linear gradient: 1min (0% B) to 10min (100% B).
4. Cyclization: The product powder was dissolved in 1mM HCl, placed in 80°C water bath. Reaction progress was monitored by UPLC until the reaction ended. Test conditions were as above mentioned.
5. Obtained positive control of the disclosure, initial sample 800828 (WT) with the original sequence, was dissolved in 20 mM NaAc pH5.0 for activity assay.

Sequences of resulting recombinant human relaxin 800828 (WT, wild-type) were as follows:

| | |
|---|---|
| chain B (WT) DSWMEEVIKLCGRELVRAQIAICGMSTWS | SEQ ID NO: 134 |
| chain A (WT) QLYSALANKCCHVGCTKRSLARFC | SEQ ID NO: 135. |

### Example 30 purification and identification of fermentation product of recombinant human relaxin 800814

Series of molecules designed in present disclosure carry amino acids with different charges at sites A₁ and B₁₄. Therefore, it is found that relaxin precursor expressed in yeast are able to be automatically cleaved within the cell, then mature relaxin molecule is secreted into the supernatant of culture medium. Intact relaxin molecule can be purified directly from the supernatant. Proceed was as follows:

### Step 1: Supernatant purified by column

Broth supernatant was purified firstly by AKTA purifier equipped with Capto MMC (GE17-5318-03) column. Column was balanced by 20mM NaAC pH4, culture supernatant was adjusted to pH 4 and sample was loaded. Eluted peaks were collected after eluting by 100mM NaHCO₃ pH 11. The pH was adjusted to 3 for subsequent RP-HPLC purification. Reverse phase preparation condition for purification: Water AutoPurifier, Kromasil 10-100-C18, 30 × 250mm; mobile phase: A solution of 0.1% TFA; B solution of ACN, flow rate at 40 ml/min, linear gradient 2min (20% B) to 15min (50% B). Eluted peaks were collected. The obtained product of interest was lyophilized, HPLC purity was 93%. By LC-MS detection, the molecular weight was 5953.0, which is consistent with the calculated molecular weight of 5952.9. The sequence of resulting recombinant human relaxin analog 800814 is as follows:

| | |
|---|---|
| chain B (D^{B14}) DSWMEEVIKLCGRDLVRAQIAICGMSTWS | SEQ ID NO: 137 |
| chain A (D^{A1}) DLYSALANKCCHVGCTKRSLARFC | SEQ ID NO: 138. |

### Step 2: Identification of disulfide bonds

The above product was dissolved in 50mM Tris pH8.0, digested with trypsin (Sigma, Cat. T1426) at 37°C overnight. After that, the reaction was terminated by addition of 3µl of 1M HCl. Fragments that were detectable by LC-MS were list in the following table. The existence of disulfide bond A24-B23 was proved, and the other two disulfide bonds were constituted by A10, A11, A15 and B11.

**Table 2. Disulfide bond identification result**

| Fragment No | Sequence | Calculated molecular weight. | Detected molecular weight |
|---|---|---|---|
| I | DSWMEEVIKLCGR DLYSALANKCCHVGCTKR | 3541.6 | 3541.7 |
| II | DLVR | 501.3 | 501.3 |
| III | SLAR | 445.5 | Not detected |
| IV | AQIAICGMSTWS FC | 1532.6 | 1532.6 |

### Example 31 Peptide fragments analysis of relaxin 800814

MS molecular weight and sequence coverage analysis of relaxin 800814 were detected by Agilent 1260 LC-6530 Q-TOF-MS instrument. The mobile phase composition in liquid phase condition:
Phase A: water (containing 0.1% formic acid); B phase: acetonitrile (containing 0.1% formic acid),
Column: Poroshell 300 SB-C8 2.1 x 75 mm 5µm, column temperature: 50 °C. Elution gradients were shown in Table 3.

**Table 3 Elution gradients**

| Time | Intact molecule | | Time | DTT reduced sample | |
|---|---|---|---|---|---|
| | phase B proportion % | Flow rate mL/min | | phase B proportion % | Flow rate mL/min |
| 0 | 5 | 0.4 | 0 | 5 | 0.4 |
| 3 | 5 | 0.4 | 3 | 5 | 0.4 |
| 10 | 95 | 0.4 | 15 | 95 | 0.4 |
| 12 | 95 | 0.4 | 18 | 95 | 0.4 |
| 12.5 | 5 | 0.4 | 18.1 | 5 | 0.4 |
| 15 | 5 | 0.4 | 21 | 5 | 0.4 |

MS conditions: ion source: AJS ESI (+), ion source parameters: Nebulizer 40 psig, Drying Gas Temp 325 °C, Gas Flow 10 l/min, Sheath Gas Temp 350 °C, Sheath Gas Flow 12 l/min. Vcap 3500 V, Fragmentor 200 V, scan range: m/z 50-3200.
Relaxin 800814 molecule weight determination: molecule weight of 5948.7996 Da, completely matching theoretical value, which proved correct expression.

Relaxin 800814 reduced peptide mapping: the protein disulfides were reduced by DTT, and sequence coverage analysis was performed to confirm the correct protein expression. Conditions were as follows: 0.1 mg/mL of sample was incubated for 2h with addition of DTT (final concentration of 20 mM), then incubated for 30min with addition of IAA (final concentration of 40 mM) in darkness. The sample after treatment was analyzed by LC-MS. The results show that the sequence of the molecule completely matches theoretical sequence, further confirms the consistency between the expressed protein molecule and the expected.

**Table 4 Results of LC-MS detection analysis**

| | | |
|---|---|---|
| relaxin 800814 chain B | DSWMEEVIKLCGRDLVRAQIAICGMSTWS | SEQ ID NO: 137 SEQ ID NO:137 |
| relaxin 800814 chain A | DLYSALANKCCHVGCTKRSLARFC | SEQ ID NO: 138 |

### Example 31 Preparation of human relaxin analog 800814PEG derivative

1. 10.0 mg of the sample recombinant human relaxin 800814 (SEQ ID NO: 19, prepared in Example 19) was accurately weighed in 50 mL reaction tube, dissolved in 5.0 mL of 0.1M CH₃COOH/CH₃COONa buffer solution (pH4.5, 4°C). Final concentration of recombinant human relaxin 800814 was 2.0 mg/mL (preparation of 0.1 M glacial acetic acid/sodium acetate: 3.7g of glacial acetic acid + 3.2g of sodium acetate +1000 mL of water).
2. 100 mg of aldehyde monomethoxy polyethylene glycol (m-PEG-CHO, 20 kDa) was accurately weighed and dissolved in 2.0 mL of tetrahydrofuran/acetonitrile (1:1) mixed solvent; the mixture was added to the above-mentioned recombinant human relaxin 800814 buffer solution (ratio of PEG to recombinant human relaxin 800814 is 3:1).
3. 0.5 mg of sodium cyanoborohydride (NaCNBH₃) was accurately weighed and dissolved in 1.0 mL of buffer solution (pH4.5), and added to the above-mentioned recombinant human relaxin 800814 buffer solution (reductive agent was introduced at a ratio of 100: 1 (reductive agent to modifying agent)).
4. The reaction mixture was maintained at room temperature (T = 25□)for 1.0 and 2.0 h, and the reaction process was detected by RP-HPLC; 1.0 mL of 10% glycine solution was added into the reaction system and incubated for 10-30 min to quench the reaction, when no more reaction product was further generated.
5. Purification: recombinant human relaxin 800814-PEG derivative (referred to as PEG-814) obtained from the reaction was separated and purified by ÄKTA purifier 10 HPLC system equipped with SP Sepharose Fast Flow cation exchange medium column (1.6cm * 18cm). The equilibration buffer solution was 20 mmol/L HAC-NaAC, with pH=4.5. After 10-fold dilution, the sample was loaded onto the column, the flow-through peak was collected and washed by the elution buffer containing 1 mol/L NaCl; the eluted peaks were collected. The flow rate was 1.0 mL/min, detection wavelength was 280 nm. The product human relaxin analog derivative PEG-814 of the present disclosure was obtained.

### Biological Evaluation

### Test Example 1: in vitro bioactivity assay of human relaxin and analogs thereof

The disclosed human relaxin analog (800814) binds to the receptor on THP-1 cells, and induces the production of cAMP in THP-1 cells. To determine the *in vitro* activity of human relaxin analog (800814), the generation of cAMP was detected. The wild-type human relaxin analog 800828 (WT) (prepared in Example 29) with original sequence was used in this test as a positive control.

Experimental cells THP-1 (ATCC, Product Number: TIB-202™) were suspension cultured at 37°C, 5% CO², and used for experiments or passage when the cells were in the logarithmic growth phase with good state. THP-1 can be passaged every 2-3 days, with a ratio of 1: 3 to 1: 4. Medium: RPMI1640, and 0.05mM b-mercaptoethanol and 10% FBS. On the day of the experiment, THP-1 cells in logarithmic growth phase were collected by centrifugation, and resuspended in DMEM/F12; the cell density was adjusted to a density of 2×10⁶ cells/ml, planted in 96-well plate (50µl each well), and incubated in 37°C incubator for 30 minutes. The human relaxin analog was 3-fold diluted by diluent (DEME/F12 + 0.2% BSA + 0.02 Polysorbate 80 +2 µM forskolin + 500µM IBMX), 50 µl of drug dilution was added in 96-well plate, mixed for 2 minutes, and incubated for 30 minutes in 37°C incubator. Then, the cells were centrifugated at 4100rpm/min for 10 minutes (at 4°C), 75µl of the supernatant was removed, 50µl of pre-cooled lysis buffer was added into each well (the whole operation was performed on ice), and incubated on ice for 20 minutes, shaking where necessary; and then the cells were centrifugated at 4100rpm/min for 10 minutes (at 4°C). The content of cAMP was detected with CAMP ELISA KIT (CELL BIOLABS).

Data obtained in the above experiment was fitted to nonlinear curve using Graphpad Prism, the EC₅₀ value (ng/ml) of CAMP in THP-1 cells which is induced by human relaxin analog (800814) was obtained. The result is shown in Table 5 below.

**Table 5: in vitro activity assay of wild-type human relaxin (800828) and human relaxin analog (800814)**

| relaxin molecule | EC50 (ng/ml)* |
|---|---|
| 800828(WT) | 3.03 |
| 800814 | 1.50 |

| | |
|---|---|
| *: EC50 is the average of three experiments | |

These results indicate that, because of changes in molecular structure, 800814 precursor molecule is able to complete digestion in cells (eliminating *in vitro* digestion purification step of expression product), furthermore *in vitro* activity of mature relaxin analog (800814) which is directly secreted into the supernatant doubles when compared with the activity of positive molecule (800828 (WT)) (EC50 increased from 3.05ng/ml to 1.50 ng/ml).

### Test Example 2 in vivo biological activity detection of human relaxin and analogs thereof

ICR mice (female, purchased from SINO-BRITSH SIPPR/BK LAB. ANIMAL LTD., CO, Certificate No.: SCXK (Shanghai) 2008-0016, 18-20 g) was used in this test; maintenance environment: SPF grade. Since purchase, ICR mice were kept in the laboratory environment for two weeks, illumination condition: 12/12-hour light/dark cycle regulation, temperature 20-25 °C; humidity 40-60%. The wild-type human relaxin analog 800828 (WT, prepared in Example 29) with original sequence was used as a positive control in this test.

One week before the experiment, each female ICR mouse was subcutaneously injected with 5µg/100µl/animal of β-estradiol (water-insoluble, slightly soluble in oil) which was mixed with olive oil. Six days later, ICR mice with body weight less than 22g were excluded, and the rest animals were equally divided into groups according to the body weight. The animals were subcutaneously injected of relaxin 800828 (WT) (6µg/100µl/mouse), 800814 (6µg/100µl/mouse) or 0.1% benzopurpurine 4B saline (solvent control group) respectively. Relaxin 800828 (WT) and 800814 were dissolved in physiological saline containing 0.1% benzopurpurin 4B. 40 hours later, the pubic bones were taken, skin and muscle were removed from the bone, the pubis width was measured under microscope; the average pubis width of the solvent control group was assigned to be 1, the relative width is represented as the ratio of width of each administration group to that of solvent control group. The results are as follows.

**Table 6. Pubic width**

| Sample | Pubic relative width |
|---|---|
| solvent control | 1 |
| Positive 800828(WT) | 1.24 |
| 800814 | 1.72 * |

| | |
|---|---|
| *: The difference between 800814 and positive molecule 800828(WT) is statistically significant. | |

These results indicate that, like 800814, the positive molecule also promotes the growth of pubic done in mice, while 800814 molecule has significantly better efficacy than positive molecule (1.72 vs 1.24, p <0.05).

### Test Example 3 In vivo half-life measurement of human relaxin and analogs thereof in rats

A total of 12 experimental SD rats (6 male and 6 female) was used in this test (purchased from SINO-BRITSH SIPPR/BK LAB. ANIMAL LTD., CO, Certificate No.: SCXK (Shanghai) 2008-0016, 160-180 g), maintanance environment: SPF grade. SD rats were kept for 3 days in laboratory environment, temperature 20-25 °C; humidity 40-60%. The wild-type human relaxin analog 800828 (WT, prepared in Example 29) with original sequence was used as a positive control in this test.

Rats were randomly divided into 3 groups, with 4 rats in each group, half males and half females. Control (saline) and human relaxin (analog 800814, WT 800828) were respectively injected into caudal vein of rats (4 rats/sample) wtih a dose of 0.5mg/kg/rat. Blood samples were taken from the rat orbital sinus at 0 h, 0.03 h, 0.08 h, 0.25 h, 0.5 h, 1 h, 1.5 h, 2 h, 4 h, 6 h and 8 h after administration. Blood samples were centrifuged, and the supernatant was collected and stored at -20 °C for further test. After blood collection, the content of relaxin in blood samples were detected with Human relaxin-2 Quantikine ELISA Kit (R&D). T_{1/2} of the test drug was calculate by T1/2 formula and EXCEL. The results are as follows:

**Table 7 In vivo half-life measurement of human relaxin and analogs thereof in rats**

| Sample | T_{1/2} (hour) |
|---|---|
| 800828(WT) | 0.87 |
| 800814 | 0.88 |

The results show that the structure change in molecule 800814 accordign to present disclosure does not affect its half-life.

### Test Example 4 Long-term in vivo activity assay of human relaxin analog derivative PEG-814

Experimental SHR female rats, 35 weeks old, weighing about 370g, were available from Beijing Weitong Lihua Experimental Animal Technology Co. Ltd., cat number: 11400700009329. Maintanance environment: SPF grade. Test sample human relaxin analog derivative PEG-814 was prepared in Example 31.

After purchase, rats, 5/cage, were kept in laboratory environment for 1 week, 12/12-hour light/dark cycle regulation, temperature 20-25 °C; humidity 50-60%. Before the experiment, basal blood pressure of SHR rats was measured 2-3 times with a non-invasive blood pressure monitor (Softron, Item No: BP-98A). Rats with stable and not less than 170 mmHg of blood pressure were selected and randomly divided into the test drug group (PEG-814) and solvent control group (saline), 10 rats in each group. Relaxin PEG-814 or saline were respectively injected into caudal vein of rats, 500 µl per time (at 30 µg/day/rat), once daily (at 15:00 p.m.), for six consecutive weeks. Blood pressure was measured once a week, body weight and blood pressure data were recorded.

Rats body weight and blood pressure changes in each group were calculated using excel statistical software, body weight and blood pressure data in test group and the solvent group were subjected to t test, blood pressure before and after administeration in both treatment group and solvent group were compared for the statistical significance and significant difference. The antihypertensive effect of PEG-814 was evaluated.

**Table 8. impact of PEG-814 on blood pressure and body weight of SHR rat**

| Index | Body Weight | | Blood pressure | |
|---|---|---|---|---|
| Group | PEG-814 | Solvent control | PEG-814 | Solvent control |
| Before administration | 362±16.54 | 355±18.97 | 203±7.16 | 203±t0.27 |
| 1 week | 363±15.79 | 355±18.12 | 193±9.78# | 198±14.12 |
| 2 weeks | 360±18.22 | 353±18.43 | 192±24.06 | 208±13.56 |
| 3 weeks | 360±16.77 | 346±17.25 | 199±14.09* | 214±18.06 |
| 4 weeks | 363±17.62 | 351±19.87 | 197±17.06 | 207±15.74 |
| 5 weeks | 361±20.32 | 353±21.64 | 199±15.8* | 214±13.96 |
| 6 weeks | 362±17.41 | 357±20.85 | 197±15.71 | 209±9.1 |
| Note: *p<0.05, vs solvent control group; | | | | |
| #p<0.05, vs before administration | | | | |

The results show that PEG-814 exhibits significant efficacy (reducing blood pressure) and long-lasting effect when administrated with once-a-day frequency; and PEG-814 shows no effect on body weight. Conventional relaxin (non-PEG-modified) requires continuous intravenous administration.

## Claims

1. A human relaxin analog, comprising chain A and chain B, the amino acid sequence of the chain A and chain B are represented by the following formula, respectively:
chain A: A₁LYSALANKCCHVGCTKRSLARFC
chain B: DSWMEEVIKLCGRB₁₄LVRAQIAICGMSTWS
wherein A₁ is selected from the group consisting of Q, D, E, and W;
wherein B₁₄ is selected from the group consisting of E, D and N;
optionally, B₁ and B₂ are absent simultaneously;
where A₁ is Q, B₁₄ is neither E nor D.

2. The human relaxin analog according to claim 1, wherein the amino acid sequence of the chain A and chain B are represented by the following formula, respectively:
chain A: A₁LYSALANKCCHVGCTKRSLARFC
chain B: DSWMEEVIKLCGRB₁₄LVRAQIAlCGMSTWS
wherein A₁ is selected from the group consisting of Q, D, E, and W; A₁ is preferably selected from the group consisting of D, E and W, and more preferably D.
wherein B₁₄ is selected from the group consisting of E, D and N;
where A₁ is Q , B₁₄ is neither E nor D.

3. The human relaxin analog according to claim 1, wherein B₁₄ is D.

4. The human relaxin analog according to claim 1, wherein the amino acid sequence of the chain A and chain B are selected from the group consisting of:
| | |
|---|---|
| chain B: DSWMEEVIKLCGRDLVRAQIAICGMSTWS | SEQ ID NO: 137 |
| chain A: DLYSALANKCCHVGCTKRSLARFC | SEQ ID NO: 138; |
| | |
| chain B: DSWMEEVIKLCGRDLVRAQIAICGMSTWS | SEQ ID NO: 137 |
| chain A: QLYSALANKCCHVGCTKRSLARFC | SEQ ID NO: 135; |
| | |
| chain B: DSWMEEVIKLCGRDLVRAQIAICGMSTWS | SEQ ID NO: 137 |
| chain A: ELYSALANKCCHVGCTKRSLARFC | SEQ ID NO: 136; |
| | |
| chain B: DSWMEEVIKLCGRNLVRAQIAICGMSTWS | SEQ ID NO: 139 |
| chain A: QLYSALANKCCHVGCTKRSLARFC | SEQ ID NO: 135; |
| | |
| chain B: WMEEVIKLCGRDLVRAQIAICGMSTWS | SEQ ID NO: 140 |
| chain A: DLYSALANKCCHVGCTKRSLARFC | SEQ ID NO: 138; |
| chain B: DSWMEEVIKLCGRELVRAQIAICGMSTWS | SEQ ID NO: 134 |
| chain A: WLYSALANKCCHVGCTKRSLARFC | SEQ ID NO: 141; |
| and | |
| chain B: DSWMEEVIKLCGRELVRAQIAICGMSTWS | SEQ ID NO: 134 |
| chain A: DLYSALANKCCHVGCTKRSLARFC | SEQ ID NO: 138. |

5. The human relaxin analog according to claim 1, wherein said chain B is linked to said chain A by linker sequence, wherein the linker sequence has 1 to 15 amino acid residues in length, preferably 2 to 8 amino acid residues.

6. The human relaxin analog according to claim 5, wherein the amino acid sequence of the linker sequence is selected from the group consisting of:
L1: KR,
L2: KRKPTGYGSRKKR, SEQ ID NO: 27,
L3: KRKPTGYGSRKR, SEQ ID NO: 28,
L4: KRGGGPRR, SEQ ID NO: 29,
L5: KRGGGPKR, SEQ ID NO: 30,
L6: KRKPTGYGSKR, SEQ ID NO: 31, and
L7: KRSLKR SEQ ID NO: 32.

7. The human relaxin analog according to claim I, wherein N terminal of said human relaxin analog is linked to signal peptide sequence, the signal peptide sequence has 4 to 15 amino acid residues in length, preferably 6 to 11 amino acid residues.

8. The human relaxin analog according to claim 7, wherein the signal peptide sequence is selected from the group consisting of:
S1: EEGEPK, SEQ ID NO: 33,
S2: EEGEPKR, SEQ ID NO: 34, and
S3: MKKNIAFLLKR, SEQ ID NO: 35.

9. A human relaxin analog derivative, wherein the derivative is obtained from PEG modification of the human relaxin analog according to any one of claims 1-8; the molecular weight of said PEG is 5 to 100 KDa, preferably 10 to 80 KDa, more preferably 15 to 45 KDa, most preferably 20 to 40 KDa; and said PEG molecule is branched-chain or linear-chain.

10. An expression precursor, expressing the human relaxin analog according to any one of claims 1-8, wherein the amino acid sequence of said expression precursor is one or more selected from the group consisting of SEQ ID NO: 1 to SEQ ID NO: 26.

11. A polynucleotide encoding the expression precursor according to claim 10.

12. An expression vector comprising the polynucleotide according to claim 11.

13. A host cell, transformed with said expression vector according to claim 12.

14. The host cell according to claim 13, wherein said host cell is bacterium, preferably *E. coli.*

15. The host cell according to claim 13, wherein said host cell is yeast, preferably *Pichia pastoris.*

16. A pharmaceutical composition, comprising or consisting of the following components:
one or more human relaxin analog(s) according to any one of claims 1-8, and/or
one or more human relaxin analog derivative(s) according to claim 9, and
one or more pharmaceutically acceptable carrier(s), diluent(s) or excipient(s).

17. An injectable solution, comprising a soluble form of the pharmaceutical composition according to claim 16.

18. Use of the human relaxin analog according to any one of claims 1-8, the human relaxin analog derivative according to claim 9, or the pharmaceutical composition according to claim 16, or the injectable solution according to claim 17, in the preparation of a medicament for treating or preventing fibrotic disease or cardiovascular disease.
